# EUROPEAN PATENT APPLICATION

(11) **EP 3 744 320 A1**
(43) Date of publication of application: **02.12.2020**
(21) Application number: 19185637.6
(22) Date of filing: 11.07.2019
(51) Int. Cl.: A61K 9/20, A61K 31/444, A61P 7/02

(54) **PHARMACEUTICAL TABLET COMPOSITION COMPRISING EDOXABAN**

(30) Priority: 29.05.2019 IN 201911021277
(71) Applicant: Alfred E. Tiefenbacher (GmbH & Co. KG), 22767 Hamburg (DE)
(72) Inventor: Rallabandi, Bala Ramesha Chary, 502 319 Telangana (IN); Joshi, Abhay Ramakant, 502 319 Telangana (IN); Chamarthi, Phanikishore Ravi, 502 319 Telangana (IN); Bandla, Srimannarayana, 502 319 Telangana (IN); Pattipati, Srikanth, 502 319 Telangana (IN); Reddy, Siva Reddy Maram, 502 319 Telangana (IN); Staver, Ruslan, 22607 Hamburg (DE); Schlehahn, Hendrik, 23843 Travenbrueck (DE)
(74) Representative: Hertin und Partner Rechts- und Patentanwälte PartG mbB

(57) **Abstract**

The invention relates to a pharmaceutical composition in the form of a tablet, comprising a) edoxaban, or a salt thereof or a hydrate of said edoxaban or edoxaban salt, as an active ingredient, b) lactose as a water-soluble filler, and c) crospovidone and sodium starch glycolate as disintegrants. The invention further relates to the pharmaceutical composition for use as a medicament in the treatment and/or prevention of a medical condition associated with unwanted blood clots, and to methods for preparing the pharmaceutical composition using wet granulation methods.

## Description

The invention relates to the field of pharmaceutical formulations of the active agent edoxaban.

The invention therefore relates to a pharmaceutical composition in the form of a tablet, comprising a) edoxaban, or a salt thereof or a hydrate of said edoxaban or edoxaban salt, as an active ingredient, b) lactose as a water-soluble filler, and c) crospovidone and sodium starch glycolate as disintegrants. The invention further relates to the pharmaceutical composition for use as a medicament in the treatment and/or prevention of a medical condition associated with unwanted blood clots, and to methods for preparing the pharmaceutical composition using wet granulation methods.

### BACKGROUND OF THE INVENTION

Edoxaban, chemically known as (N'-(5-chloropyridin-2-yl)-N-[(1S,2R,4S)-4-(dimethylcarbamoyl)-2-[(5-methyl-6,7-dihydro-4H-[1,3]thiazolo[5,4-c]pyridine-2-carbonyl)amino]cyclohexyl]oxamide), often used as edoxaban tosylate monohydrate, is an oral anticoagulant drug which acts as a direct factor Xa inhibitor.

Factor Xa is a blood coagulation factor that is responsible for the initiation of the coagulation cascade. Factor Xa cleaves prothrombin to its active form thrombin, which acts to convert soluble fibrinogen to insoluble fibrin and to activate platelets. Stabilization of the platelet aggregation ultimately leads to clot formation. Edoxaban inhibits free factor Xa and its prothrombinase activity and therefore inhibits thrombin-induced platelet aggregation and reduces thrombin generation and thrombus formation.

Edoxaban is approved as 15 mg, 30 mg, and 60 mg film-coated tablets by the European Medicines Agency (EMA), the U. S. Food and Drug Administration (FDA), and the Japanese Pharmaceuticals and Medical Devices Agency (PMDA) and is marketed under the trade names Savaysa® (US) and Lixiana® (EU, JP) by Daiichi Sankyo.

According to the European public assessment report (EPAR) summary, Lixiana® is an anticoagulant medicine that prevents blood clotting, used in adults:
- to prevent stroke (caused by blood clots in the brain) and systemic embolism (SE, blood clots in other organs) in patients with non-valvular atrial fibrillation (NVAF, irregular rapid contractions of the upper chambers of the heart). It is used in patients who have one or more risk factors, such as having had a previous stroke, high blood pressure, diabetes, heart failure or being 75 years old or over; and
- to treat deep-vein thrombosis (DVT, a blood clot in a deep vein, usually in the leg) and pulmonary embolism (a clot in a blood vessel supplying the lungs), and to prevent DVT and pulmonary embolism from re-occurring.

Edoxaban has also been approved in the US and Japan for these indications. Additionally, it is approved in Japan for the prevention of venous thromboembolisms (VTE) following lower-limb orthopedic surgery.

EP1405852 (Daiichi Sankyo) describes the chemical structure of edoxaban and analogues thereof. WO2011/115066 (Daiichi Sankyo) describes a process for the preparation of polymorphic form II of edoxaban tosylate monohydrate. However, the commercial polymorph employed is typically form I, which is the most thermodynamically stable form.

Various formulations of edoxaban have been disclosed in the art.

The commercial formulation of the reference product Savaysa® and Lixiana® is dosis-proportional for all three approved strengths with the tablet core weight of 100 mg, 200 mg, and 400 mg, correspondingly, and contains edoxaban tosylate monohydrate (equivalent to 15 mg, 30 mg, and 60 mg edoxaban free base, 15 % by weight of core based on free base content), mannitol, pregelatinised starch, crospovidone, hydroxypropylcellulose and magnesium stearate.

WO2008/129846 (Daiichi Sankyo) discloses commercial formulations of edoxaban that contain edoxaban tosylate monohydrate, mannitol, pregelatinised starch, crospovidone, hydroxypropylcellulose and magnesium stearate. The commercial formulation of 30 mg tablet is disclosed therein. Of note, is that the compositions described therein exhibit variable dissolution properties. This is observed by comparing Figures 3 and 5 of WO2008/129846. Composition L (comprising lactose) is shown in Figure 3 (0.1 N hydrochloric acid dissolution medium) to exhibit slower dissolution over Composition M (comprising mannitol). However, Figure 5 (phosphate buffer pH 6.8 dissolution medium) shows the opposite results, in which Composition L shows faster dissolution compared to Composition M. The compositions of the prior art therefore exhibit variable dissolution properties under different dissolution conditions.

WO2011/115067 (Daiichi Sankyo) discloses the same edoxaban compositions and methods for formulating edoxaban comprising a wet granulation process. The manufacturing process of the commercial composition of edoxaban under low moisturization water conditions is disclosed (table 4, examples B-1, B-2, B-3). The disclosed wet granulation process is carried out under low moisturization water conditions in order to keep the water content of the granules below 10% during the granulation step. It is shown that the moisture content during the wet granulation step and also the residual water content has a high influence on the dissolution of the resulting tablets.

WO2011/115067 also discloses that the composition according to WO2008/129846 produced under high moisturization water conditions (examples A-1, A-2, A-3) shows high variation (RSD) in dissolution when assessing single tablets. For example, Figure 1 for A-2, shows that 2 of 6 tablets have less than 50% drug release at the end point of the dissolution profile in pH 6.8 media. As can be seen in figure 3, the RSD for this example A-2 is very high being more than 20%. The disclosure of WO2011/115067 therefore potentially provides some explanation for the variable dissolution seen in WO2008/129846.

WO2010/147169 (Daiichi Sankyo) discloses edoxaban 30 mg tablets wherein the content of edoxaban is less than 10% by weight with respect to the total weight of the composition. However, such an approach will lead to much larger tablets of 60 mg tablets compared to the size of the reference product Lixiana, and is not acceptable from regulatory point of view, e.g. in view of US FDA guidance "Size, Shape, and Other Physical Attributes of Generic Tablets and Capsules" (dated June 18, 2015),

WO2013/22059 (Daiichi Sankyo) discloses edoxaban tablets and capsule formulations comprising one or more of carmellose and fumaric acid.

WO2016/20080 (Sandoz) discloses edoxaban compositions comprising multiple water soluble vinylpyrrolidone polymers as binders, selected from povidone and copovidone, and a cellulose ether, without a sugar alcohol.

WO2017/107857 (Jiangsu Hengrui) discloses edoxaban formulations with two or more cellulose derivatives as binders.

WO2010/71164 (Daiichi Sankyo) discloses specific dosage regimes for administering edoxaban in the treatment of thromboembolism based on non-valvular atrial fibrillation.

Edoxaban is a poorly soluble compound for which various attempts at developing suitable formulations have been undertaken, as described above. Edoxaban exhibits high solubility in a strong acidic aqueous solution, but its solubility decreases in a neutral pH aqueous solution. Wet granulation manufacturing processes have shown some success in producing edoxaban formulations with acceptable dissolution. However, often multiple water-soluble or cellulose derivative binders were required, or large amounts of sugar alcohol or relatively low amounts of active agent employed, in order to achieve sufficient dissolution profiles.

Considering the inherent difficulties with the low solubility of edoxaban and variability of the dissolution of the tablets, novel means are required for providing an edoxaban formulation in which the composition retains acceptable hardness (in the case of tablets) and disintegration properties, whilst achieving good and reliable dissolution of therapeutically relevant amounts of edoxaban across various dissolution conditions.

### SUMMARY OF THE INVENTION

In light of the prior art the technical problem underlying the invention was the provision of improved or alternative means for pharmaceutical compositions comprising edoxaban with acceptable hardness, disintegration, stability and dissolution properties. A further object of the invention is to provide improved or alternative means for pharmaceutical compositions comprising edoxaban with more consistent dissolution properties in various dissolution media compared to the compositions of the prior art.

In providing a solution to this problem, the invention seeks to avoid the disadvantages of the prior art.

This problem is solved by the features of the independent claims. Preferred embodiments of the present invention are provided by the dependent claims.

Therefore, the invention relates to a pharmaceutical composition in the form of a tablet, comprising a) edoxaban, or a salt thereof or a hydrate of said edoxaban or edoxaban salt, as an active ingredient, b) lactose as a water-soluble filler, and c) crospovidone and sodium starch glycolate as disintegrants.

Surprisingly, the compositions of the present invention show excellent dissolution properties, whilst providing sufficient tablet hardness. Furthermore, the compositions of the invention described herein disintegrate effectively and show excellent chemical stability upon storage. In preferred embodiments, the compositions of the present invention comprise, in contrast to the prior art, a single binder and the presence of two disintegrants. The presence of the two disintegrants crospovidone and sodium starch glycolate in combination with lactose enable the use of various additional excipients without significant loss in the dissolution properties described herein. The dissolution of the tablet compositions of the present invention is also more consistent in varying dissolution media compared to compositions of the prior art. The present invention also allows a straightforward manufacturing process, preferably employing wet granulation, to produce edoxaban tablets with the beneficial properties described herein.

As exemplified in the experimental examples below, the use of a single disintegrant crospovidone is often insufficient in order to achieve the desired dissolution properties. Additionally, the use of a single disintegrant SSG, even in the presence of pregelatinized starch or additional amounts of lactose, appears insufficient in order to achieve the desired dissolution properties. Employing the combination of lactose as a water-soluble filler and crospovidone and sodium starch glycolate as disintegrants, does not lead to significantly improved disintegration time, however the dissolution of the active ingredient is significantly improved. This represents a surprising and unexpected technical effect of the invention, that the combined use of the disintegrants of the invention improves dissolution, without necessarily effecting disintegration.

A skilled person would not usually consider the addition of multiple disintegrants in tablet formulation and would certainly not have expected that the combination of the disintegrants of the invention could leave disintegration of the tablet essentially unaffected whilst improving active agent dissolution. The invention is therefore based, in preferred embodiments, on the surprising finding of the disintegrant combination enabling improved dissolution. In some embodiments, the presence of PGS further leads to improved dissolution with the combined disintegrants of the invention.

As outlined above, WO2008/129846 discloses commercial formulations of edoxaban that contain edoxaban tosylate monohydrate, mannitol, pregelatinised starch, crospovidone, hydroxypropylcellulose and magnesium stearate. WO2008/129846 further discloses that lactose is inferior to mannitol as a filler. However, lactose is typically associated with lower cost when formulating tablets and, without being bound by theory, can lead to smaller particle sizes during granulation approaches, potentially helping dissolution. Furthermore, mannitol has been shown to exhibit unwanted side effects in some cases similar to sorbitol and other polyalcohol fillers, as it can impact negatively on gastrin emptying rate and intestinal transit time. Due to osmotic load in the small intestine induced by a sugar alcohol like mannitol and sorbitol, it can negatively influence the absorption of poorly permeable active pharmaceutical ingredients and thus reduce the bioavailability of drugs.

In contrast, lactose is largely regarded as a filler with extremely low rates of side effects and has no reaction with most drugs. Tablets compressed from lactose granules often show excellent uniformity of weight after production.

In order to explore the possibility of employing lactose as a filler in edoxaban tablets, the inventors assessed various compositions comprising lactose, as shown in the examples below.

Surprisingly, tablets with good dissolution properties have been formulated using lactose, in contrast to the assertions in WO2008/129846. As shown in the examples below, the lactose-based formulations of the present invention surprisingly show improved dissolution properties over mannitol comprising tablets, as described in the prior art.

In some embodiments, lactose is an essential component of the composition. In some embodiments, the lactose may selected from the group consisting of lactose monohydrate, anhydrous lactose, spray dried lactose and co-processed lactose. Examples of co-processed lactose are Ludipress® (lactose monohydrate 93 wt.%, 3.5 wt.% povidone (Kollidon®30) and 3.5 wt.% crospovidone (Kollidon® CL); Cellactose® (75% lactose and 25% microcrystalline cellulose MCC); Starlac® (85% lactose and 15% starch) and Combilac® (70% lactose, 20% MCC and 10% corn starch).

The use of lactose is advantageous, as lactose is in particular cheaper than other excipients and thus allows a more economic production of pharmaceutical tablet compositions. Furthermore, the use of lactose is advantageous, as a variety of commercially available grades (lactose monohydrate, anhydrous lactose and spray-dried lactose) exists, which enables the skilled person to choose the best fitting grade for the present situation. Accordingly, the skilled person is enabled with respect to all aspects of the present invention to select the best fitting lactose excipient based on the required characteristics for tablet compression. For example, in case the lactose excipient shall also facilitate non-sticking at the compression facilities, lactose comprising a comparatively large particle size will be selected. In case lactose monohydrate is used, fine grades are usually used for the wet granulation process, as they permit better mixing with other excipients.

Therefore, the combination of lactose as a water-soluble filler and crospovidone and sodium starch glycolate as disintegrants provides a tablet with sufficient hardness, good chemical stability, sufficient disintegration and very good dissolution of the active agent.

In a preferred embodiment the active ingredient is edoxaban tosylate monohydrate.

In one embodiment, the active ingredient has a particle size distribution (D90 by volume) of less than or equal to 40 µm.

As an additional advantage of the invention, the combination of the two disintegrants crospovidone and sodium starch glycolate with lactose enables the use of different grades of active agent. Previous attempts at formulating tablets with good dissolution properties involved the use of very low particle size distributions of active ingredient. According to some embodiments, the present invention enables edoxaban with a particle size distribution of up to 40 µm without significant loss in the solubility of the active agent.

In one embodiment, the active ingredient has a particle size distribution (D90 by volume) of 1 to 40 µm. In one embodiment, the active ingredient has a particle size distribution (D90 by volume) of 3 to 15 µm, preferably 5 to 10 µm, for example about 5, 6, 7, 8, 9 or 10. In one embodiment, the active ingredient has a particle size distribution (D90 by volume) of 3 to 15 µm. In one embodiment, the active ingredient has a particle size distribution (D90 by volume) of 20 to 40 µm, preferably 30 to 40 µm.

In one embodiment the composition comprises additionally a binder, an additional filler and a lubricant, or wherein the composition comprises additionally a binder and a lubricant without an additional binder. In one embodiment, the tablet comprises one binder.

In one embodiment, the binder is hydroxypropylcellulose. In one embodiment, the additional filler is pregelatinized starch. In one embodiment, the lubricant is magnesium stearate. In one embodiment, the binder is hydroxypropylcellulose, the additional filler is pregelatinized starch, and/or the lubricant is magnesium stearate.

In one embodiment, the composition is prepared by wet granulation. As shown in the examples below, wet granulation enables a straightforward manufacture with flexibility as to which components are added in the intra- or extragranular phase. Furthermore, as shown in the examples below, wet granulation process enables tablets with improved dissolution properties of the active agent compared to direct compression methods of manufacture.

The method of manufacture employed, here preferably wet granulation, leads directly to structural features of the tablet produced by such a method. A skilled person can determine whether tablets were generated via direct compression, wet or dry granulation using established techniques. The definition of the tablet by the wet granulation method of manufacture has a clear and reliable effect on the structure of the tablet and physical and functional properties of the tablet. In some embodiments, features related to the method of manufacture therefore apply to embodiments of the tablet as such, and vice versa.

In one embodiment, the wet granulation comprises preferably fluid bed granulation.

When considering a) is edoxaban, or a salt thereof or a hydrate of said edoxaban or edoxaban salt, b) is lactose and c) is crospovidone and sodium starch glycolate, in one embodiment the wet granulation comprises granulation of a blend of at least components a) and b) and optionally c) to produce granules, optional addition of one or more components c) to the granules, such that both crospovidone and sodium starch glycolate are present, and compression of the granules to a tablet.

In one embodiment, crospovidone and sodium starch glycolate are extragranular (added after wet granulation).

In one embodiment, an additional filler and a lubricant, preferably those described above, are extragranular.

In one embodiment, the tablet is coated, preferably wherein the coating is a film coating comprising hydroxypropyl methyl cellulose (HPMC, hypromellose) and/or polyethylene glycol (PEG, Macrogol). The coating of the tablet has been carried out in prior art tablets and represent a preferred embodiment. A skilled person can determine appropriate coating materials and methods for coating without undue effort.

In one embodiment, the pharmaceutical composition as described herein comprises:
a. edoxaban, or a salt thereof or a hydrate of said edoxaban or edoxaban salt, preferably edoxaban tosylate monohydrate, present in an amount of 10-30 wt%, preferably 15-25 wt%, more preferably about 18-22 wt%,
b. lactose, preferably lactose monohydrate, present in an amount of 20-70 wt%, preferably 30-60 wt%, more preferably about 35-55 wt%,
c. crospovidone, present in an amount of 0.5-25 wt%, preferably 1-10 wt%, more preferably about 1.5-8 wt%,
d. sodium starch glycolate, present in an amount of 1-25 wt%, preferably 2-20 wt%, more preferably about 4-15%,
based on the total weight of all components of the tablet.

In one embodiment, the pharmaceutical composition as described herein comprises additionally:
e. lubricant, preferably magnesium stearate, present in an amount of 0.1-5 wt%, preferably 0.25-2.5 wt%, more preferably about 0.5-2 wt%,
f. pregelatinized starch, present in an amount of 0-30 wt%, preferably 4-20 wt%, more preferably about 5-18 wt%,
g. hydroxypropylcellulose, present in an amount of 1-5 wt%, preferably 2-4 wt%, more preferably about 2.5-3.5 wt%,
h. a coating agent, preferably comprising hydroxypropyl methyl cellulose and/or polyethylene glycol (PEG, Macrogol), present in an amount of 1-10 wt%, preferably 3-7 wt%, more preferably about 4-6 wt%,
based on the total weight of all components of the tablet.

In one embodiment, the pharmaceutical composition as described herein comprises:
a. edoxaban, or a salt thereof or a hydrate of said edoxaban or edoxaban salt, preferably edoxaban tosylate monohydrate, present in an amount of 10-30 wt%, preferably 15-25 wt%, more preferably about 18-22 wt%,
b. lactose, preferably lactose monohydrate, present in an amount of 20-70 wt%, preferably 30-60 wt%, more preferably about 35-55 wt%,
c. crospovidone, present in an amount of 0.5-25 wt%, preferably 1-10 wt%, more preferably about 1.5-8 wt%,
d. sodium starch glycolate, present in an amount of 1-25 wt%, preferably 2-20 wt%, more preferably about 4-15%,
e. lubricant, preferably magnesium stearate, present in an amount of 0.1-5 wt%, preferably 0.25-2.5 wt%, more preferably about 0.5-2 wt%,
f. hydroxypropylcellulose, present in an amount of 1-5 wt%, preferably 2-4 wt%, more preferably about 2.5-3.5 wt%, and
g. a coating agent, preferably comprising hydroxypropyl methyl cellulose and/or polyethylene glycol (PEG, Macrogol), present in an amount of 1-10 wt%, preferably 3-7 wt%, more preferably about 4-6 wt%,
based on the total weight of all components of the tablet.

In a preferred embodiment, the pharmaceutical composition as described herein comprises the components, preferably in those amounts mentioned, also considering the preferred ranges, for the composition **EDO/E-16** as described below.

All values provided above, for example the specific preferred values for each component, may vary by +/- 2 wt%, or by +/- 1 wt%.

The below embodiments may also be considered to encompass further embodiments of the invention in which the indicated amounts of the components are employed. These embodiments are not limited by total weight of the compositions, but in some embodiments by the wt% values of each component and/or the presence of each component in the intra- or extragranular phase or in the coating, without limitation to absolute weight.

**Embodiments based on EDO/E-1 of Edoxaban 60 mg core tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate | API | 83.230 | 20.81% | 15-25 |
| 2 | Lactose Monohydrate | Water-soluble Filler | 179.770 | 44.94% | 40-50 |

| **Stage-B (Binder solution):** | | | | | |
|---|---|---|---|---|---|
| 1 | Hydroxypropylcellulose | Binder | 12.000 | 3.00% | 1-5 |
| 2 | Water | Solvent for binder solution | q.s. | | |

| **Stage-C (Blending & Lubrication):** | | | | | |
|---|---|---|---|---|---|
| 1 | Crospovidone | Superdisintegrant | 80.000 | 20.00% | 15-25 |
| 2 | Sodium Starch Glycolate | Superdisintegrant | 40.000 | 10.00% | 5-15 |
| 3 | Magnesium Stearate | Lubricant | 5.000 | 1.25% | 0.5-2 |
| ***Total weight of Core Tablets*** | | | **400.000** | | |

**Embodiments based on EDO/E-2 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate | API | 83.230 | 19.82% | 15-25 |
| 2 | Lactose Monohydrate | Water-soluble Filler | 195.770 | 46.61% | 30-60 |

| **Stage-B (Binder solution):** | | | | | |
|---|---|---|---|---|---|
| 1 | Hydroxypropylcellulose | Binder | 12.000 | 2.86% | 1-5 |
| 2 | Water | Solvent for binder solution | q.s. | | |

| **Stage-C (Blending & Lubrication):** | | | | | |
|---|---|---|---|---|---|
| 1 | Sodium Starch Glycolate | Superdisintegrant | 84.000 | 20.00% | 15-25 |
| 2 | Crospovidone | Superdisintegrant | 22.000 | 5.24% | 2-10 |
| 3 | Magnesium Stearate | Lubricant | 3.000 | 0.71% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | | **420.000** | | |

**Embodiments based on EDO/E-3 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate | API | 83.230 | 19.82% | 15-25 |
| 2 | Lactose Monohydrate | Water-soluble Filler | 194.770 | 46.37% | 30-60 |
| 3 | Crospovidone | Superdisintegrant | 10.000 | 2.38% | 1-5 |

| **Stage-B (Binder solution):** | | | | | |
|---|---|---|---|---|---|
| 1 | Hydroxypropylcellulose | Binder | 12.000 | 2.86% | 1-5 |
| 2 | Water | Solvent for binder solution | q.s. | | |

| **Stage-C (Blending & Lubrication):** | | | | | |
|---|---|---|---|---|---|
| 1 | Pregelatinized Starch | Filler-Disintegrant | 72.000 | 17.14% | 12-22 |
| 2 | Sodium Starch Glycolate | Superdisintegrant | 24.000 | 5.71% | 2-10 |
| 3 | Magnesium Stearate | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | | **420.000** | | |

**Embodiments based on EDO/E-4, EDO/E-5, EDO/E-6, EDO/E-7, and EDO/E-8 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate | API | 83.830 | 19.96% | 15-25 |
| 2 | Lactose Monohydrate | Water-soluble Filler | 194.170 | 46.23% | 30-60 |
| 3 | Crospovidone | Superdisintegrant | 10.000 | 2.38% | 1-5 |

| **Stage-B (Binder solution):** | | | | | |
|---|---|---|---|---|---|
| 1 | Hydroxypropylcellulose | Binder | 12.000 | 2.86% | 1-5 |
| 2 | Water | Solvent for binder solution | q.s. | | |

| **Stage-C (Blending & Lubrication):** | | | | | |
|---|---|---|---|---|---|
| 1 | Pregelatinized Starch | Filler-Disintegrant | 72.000 | 17.14% | 12-22 |
| 2 | Sodium Starch Glycolate | Superdisintegrant | 24.000 | 5.71% | 1-10 |
| 3 | Magnesium Stearate | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | ***Tablets*** | **420.000** | | |

**Embodiments based on EDO/E-11 of Edoxaban 60 mg core tablets:**

| **Batch Number EDO/E-11** | | | **EDO/E-11** | **%wt** | **Preferred range %wt** |
|---|---|---|---|---|---|
| **Stage-A (Dry mix):** | | | | | |
| 1 | Edoxaban Tosylate Monohydrate* *In-House* | API | 83.830 | 19.96% | 15-25 |
| 2 | Lactose Monohydrate *Ph.Eur.* (Granulac® 200) | Water-soluble Filler | 194.170 | 46.23% | 40-50 |
| 3 | Crospovidone *Ph. Eur.* (Polyplasdone® XL-10) | Superdisintegrant | 10.000 | 2.38% | 1-5 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Pregelatinized Starch (Starch 1500®) | Filler-Disintegrant | 62.000 | 14.76% | 10-20 |
| 2 | Sodium Starch Glycolate (Explotab® CLV) | Superdisintegrant | 34.000 | 8.10% | 5-12 |
| 3 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | | **420.000** | | |

**Embodiments based on EDO/E-12 of Edoxaban 60 mg core tablets:**

| **Batch Number** | | | **EDO/E-11** | **%wt** | **Preferred range %wt** |
|---|---|---|---|---|---|
| **Stage-A (Dry mix):** | | | | | |
| 1 | Edoxaban Tosylate Monohydrate* *In-House* | API | 83.830 | 19.96% | 15-25 |
| 2 | Lactose Monohydrate *Ph.Eur.* (Granulac® 200) | Water-soluble Filler | 194.170 | 46.23% | 40-50 |
| 3 | Crospovidone *Ph. Eur.* (Polyplasdone® XL-10) | Superdisintegrant | 10.000 | 2.38% | 1-5 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Pregelatinized Starch (Starch 1500®) | Filler-Disintegrant | 62.000 | 11.43% | 5-15 |
| 2 | Sodium Starch Glycolate (Explotab® CLV) | Superdisintegrant | 34.000 | 11.43% | 5-15 |
| 3 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | | **420.000** | | |

**Embodiments based on EDO/E-13 of Edoxaban 60 mg core tablets:**

| **Batch Number** | | | **EDO/E-11** | **%wt** | **Preferred range %wt** |
|---|---|---|---|---|---|
| **Stage-A (Dry mix):** | | | | | |
| 1 | Edoxaban Tosylate Monohydrate* *In-House* | API | 83.830 | 19.96% | 15-25 |
| 2 | Lactose Monohydrate *Ph.Eur.* (Granulac® 200) | Water-soluble Filler | 194.170 | 46.23% | 40-50 |
| 3 | Crospovidone *Ph. Eur.* (Polyplasdone® XL-10) | Superdisintegrant | 10.000 | 2.38% | 1-5 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Pregelatinized Starch (Starch 1500®) | Filler-Disintegrant | 62.000 | 13.33% | 8-20 |
| 2 | Sodium Starch Glycolate (Explotab® CLV) | Superdisintegrant | 34.000 | 9.52% | 5-15 |
| 3 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry | Coating | 20.000 | 4.76% | 2-8 |
| 2 | Water | Solvent for coating dispersion | q.s. | | |
| ***Total weight of Film-Coated Tablets*** | | | **420.000** | | |

**Embodiments based on EDO/E-14 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate *In-House* | API | 83.830* | 19.96% | 15-25 |
| 2 | Lactose Monohydrate *Ph.Eur.* (Granulac® 200) | Water-soluble Filler | 224.170 | 53.37% | 40-60 |
| 3 | Crospovidone *Ph. Eur.* (Polyplasdone® XL-10) | Superdisintegrant | 10.000 | 2.38% | 1-5 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Pregelatinized Starch (Starch 1500®) | Filler-Disintegrant | 42.000 | 10.00% | 5-15 |
| 2 | Sodium Starch Glycolate (Explotab® CLV) | Superdisintegrant | 24.000 | 5.71% | 1-10 |
| 3 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry® 03F520440 Yellow | Coating | 20.000 | 4.76% | 2-8 |
| 2 | Water, Purified *Ph. Eur.* | Solvent for coating dispersion | q.s. | | |
| ***Total weight of Film-Coated Tablets*** | | | **420.000** | | |

**Embodiments based on EDO/E-15 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate *In-House* | API | 83.833* | 19.96% | 15-25 |
| 2 | Lactose Monohydrate *Ph.Eur.* (Granulac® 200) | Water-soluble Filler | 169.167 | 40.28% | 30-50 |
| 3 | Crospovidone *Ph. Eur.* (Polyplasdone® XL-10) | Superdisintegrant | 10.000 | 2.38% | 1-5 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Pregelatinized Starch (Starch 1500®) | Filler-Disintegrant | 72.000 | 17.14% | 10-25 |
| 2 | Sodium Starch Glycolate (Explotab® CLV) | Superdisintegrant | 24.000 | 5.71% | 1-10 |
| 3 | Crospovidone *Ph. Eur.* (Polyplasdone® XL) | Superdisintegrant | 25.000 | 5.95% | 1-10 |
| 4 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry® 03F520440 Yellow | Coating | 20.000 | 4.76% | 2-8 |
| 2 | Water, Purified *Ph. Eur.* | Solvent for coating dispersion | q.s. | | |
| ***Total weight of Film-Coated Tablets*** | | | **420.000** | | |

**Embodiments based on EDO/E-16 of Edoxaban 60 mg film-coated tablets:**

| **S. No.** | **Ingredients** | **Function** | **mg/tab** | **%wt** | **Preferred range %wt** |
|---|---|---|---|---|---|
| **Stage-A (Dry mix):** | | | | | |
| 1 | Edoxaban Tosylate Monohydrate *In-House* | API | 83.833* | 19.96% | 15-25 |
| 2 | Lactose Monohydrate *Ph.Eur.* (Granulac® 200) | Water-soluble Filler | 194.167 | 46.23% | 30-60 |

| **Stage-B (Binder solution):** | | | | | |
|---|---|---|---|---|---|
| 1 | Hydroxypropylcellulose *Ph. Eur.* (Klucel® ELF) | Binder | 12.000 | 2.86% | 1-5 |
| 2 | Water, Purified *Ph. Eur.* | Solvent for binder solution | q.s. | | |

| **Stage-C (Blending & Lubrication):** | | | | | |
|---|---|---|---|---|---|
| 1 | Sodium Starch Glycolate (Explotab® CLV) | Superdisintegrant | 84.000 | 20.00% | 10-30 |
| 2 | Crospovidone *Ph. Eur.* (Polyplasdone® XL) | Superdisintegrant | 22.000 | 5.24% | 1-10 |
| 3 | Magnesium Stearate *Ph. Eur.* | Lubricant | 4.000 | 0.95% | 0.1-2 |
| ***Total weight of Core Tablets*** | | | **400.000** | | |

| **Stage-D (Film-Coating):** | | | | | |
|---|---|---|---|---|---|
| 1 | Opadry® 03F520440 Yellow | Coating | 20.000 | 4.76% | 2-8 |
| 2 | Water, Purified *Ph. Eur.* | Solvent for coating dispersion | q.s. | | |
| ***Total weight of Film-Coated Tablets*** | | | **420.000** | | |

In some embodiments, the composition may comprise 15 mg, 30 mg or 60 mg of active ingredient.

In one embodiment, the tablet shows at least 75% dissolution of the active ingredient after 45 minutes using method 2 of the dissolution test of the US Pharmacopeia (USP), with a paddle speed of 50 revolutions per minute in 900 mL of acetate buffer at pH 4.5. Accordingly, the tablets of the present invention show good dissolution properties and fulfill the requirements of the above-mentioned regulation. Further evidence for and descriptions of the dissolution properties of the active ingredient are provided herein, in particular in the examples below.

In a further aspect, the invention relates to a pharmaceutical composition as described herein for use as a medicament in the treatment and/or prevention of a medical condition associated with unwanted blood clots.

The invention therefore also relates to corresponding methods of treatment, for example a method of treating a subject with a medical condition associated with unwanted blood clots, the method comprising administering a therapeutically effective amount of a pharmaceutical composition as described herein to the subject.

In further embodiments the medical condition associated with unwanted blot clots are selected from the list consisting of stroke, systemic embolism, non-valvular atrial fibrillation, venous thromboembolism, deep-vein thrombosis and pulmonary embolism. A skilled person is capable of determining which conditions are associated with unwanted blood clots and determining an effective dose based on common knowledge in the art. According to the present invention, the term "unwanted blood clot" refers to any blood clot that causes, increases the risk of, or is associated in any way with a medical condition or pathology that is detrimental or could be detrimental to the health of a subject.

In a further aspect, the invention relates to a method of preparing a pharmaceutical composition in the form of a tablet, the method comprising wet granulation.

In some embodiments, the method comprises fluid bed granulation.

In some embodiments, the method comprises granulation, preferably fluid bed granulation, of a blend of at least components a) and b) and optionally c) to produce granules, optional addition of one or more components c) to the granules, such that both crospovidone and sodium starch glycolate are present, and compression of the granules to a tablet. This embodiment refers to the components a) is edoxaban, or a salt thereof or a hydrate of said edoxaban or edoxaban salt, b) is lactose and c) is crospovidone and sodium starch glycolate.

In one embodiment, the method of preparing a pharmaceutical composition in the form of a tablet comprises:
a. blending edoxaban, or a salt thereof or a hydrate of said edoxaban or edoxaban salt, preferably edoxaban tosylate monohydrate, with lactose, preferably lactose monohydrate, and optionally a disintegrant selected from crospovidone and sodium starch glycolate to produce a blend;
b. wet granulating the blend of a. with a binder, preferably hydroxypropylcellulose, to produce granules;
c. blending the granules of b. with one or more of crospovidone and sodium starch glycolate, such that both crospovidone and sodium starch glycolate are present from steps a. and/or c., and optionally an additional filler, preferably pregelatinized starch, and optionally a lubricant, preferably magnesium stearate;
d. optionally lubricating the granules of c. by mixing with a lubricant, preferably magnesium stearate, such that at least one lubricant is present from steps c. and/or d.; and
e. compression of the granules to a tablet.

In one embodiment, the method comprises additionally coating the tablet with a film coating, preferably comprising hydroxypropyl methyl cellulose (HPMC, hypromellose) and/or polyethylene glycol (PEG, Macrogol).

The features above with respect to the method of preparing a pharmaceutical composition have a structural and functional outcome on the tablets of the present invention, such that the tablets can in some embodiments be described by features of or derived from the method of preparing, and vice versa.

### DETAILED DESCRIPTION OF THE INVENTION

Edoxaban (INN), chemically known as (N'-(5-chloropyridin-2-yl)-N-[(1S,2R,4S)-4-(dimethylcarbamoyl)-2-[(5-methyl-6,7-dihydro-4H-[1,3]thiazolo[5,4-c]pyridine-2-carbonyl)amino]cyclohexyl]oxamide), is an oral anticoagulant drug which acts as a direct factor Xa inhibitor. Edoxaban is approved as 15 mg, 30 mg, and 60 mg film-coated tablets by the European Medicines Agency (EMA), the U. S. Food and Drug Administration (FDA), and the Japanese Pharmaceuticals and Medical Devices Agency (PMDA), and is marketed under the trade names Savaysa® (US) and Lixiana® (EU, JP) by Daiichi Sankyo

The chemical structure of edoxaban is:

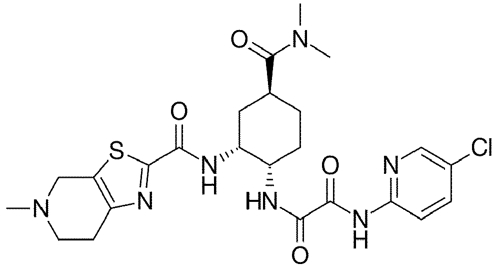

The chemical structure of edoxaban tosylate monohydrate is represented as:

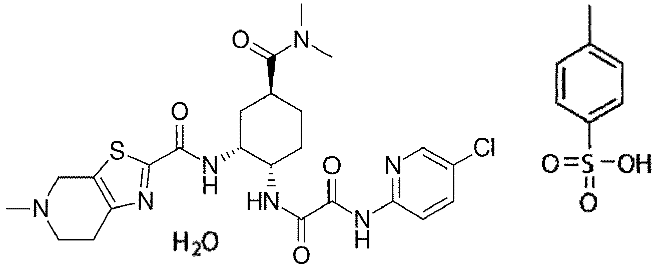

According to the summary of product characteristics (SmPC), the regulatory approved indication in the European Union (EU) is for prevention of stroke and systemic embolism in adult patients with nonvalvular atrial fibrillation (NVAF) with one or more risk factors, such as congestive heart failure, hypertension, age ≥ 75 years, diabetes mellitus, prior stroke or transient ischaemic attack (TIA). The second indication approved in EU is the treatment of deep vein thrombosis (DVT) and pulmonary embolism (PE), and prevention of recurrent DVT and PE in adults.

According to FDA label of Savaysa®, edoxaban is approved in US:
- to reduce the risk of stroke and systemic embolism (SE) in patients with nonvalvular atrial fibrillation (NVAF);
- for the treatment of deep vein thrombosis (DVT) and pulmonary embolism (PE) following 5 to 10 days of initial therapy with a parenteral anticoagulant.

The therapeutic indications approved in Japan by PMDA are:
- reduction of the risk of ischaemic stroke and systemic embolism in patients with non-valvular atrial fibrillation;
- treatment and prophylaxis of the relapse of venous thromboembolism (deep vein thrombosis and pulmonary thromboembolism);
- reduction of the risk of venous thromboembolism in patients undergoing any of the following orthopedic surgeries for the lower limbs: total knee replacement, total hip replacement, and hip fracture surgery.

These medical indications represent preferred embodiments of the medical use of the invention.

According to the European public assessment report (EPAR) of the reference product Lixiana®, the used active pharmaceutical ingredient (API) is edoxaban tosylate monohydrate. The API is a white to pale yellowish-white non-hygroscopic crystalline powder, which is practically insoluble in isopropanol and ethyl acetate, slightly soluble in water, ethanol and acetonitrile, soluble in methanol and freely soluble in DMSO and N,N-dimethylformamide.

The API exists in two polymorphic forms, form I (thermodynamically most stable form) and form II. The crystalline form consistently produced by the most commonly used synthetic route is form I. In addition, the results from the stability studies conducted on API show no evidence of conversion between form I to form II.

When administered in vivo, edoxaban is absorbed with peak plasma concentrations within 1 - 2 hours. The absolute bioavailability is approximately 62%. Edoxaban is primarily absorbed (approximately 85%) in the upper gastrointestinal tract and is poorly soluble at pH of 6.0 or higher.

The term "active ingredient" or "API" herein refers to a pharmaceutically active molecule (e.g. edoxaban) as well as its pharmaceutically acceptable and therapeutically active salts, hydrates, esters, amides, prodrugs, metabolites, enantiomers, polymorphs, analogs, etc. that induce a desired pharmacological or physiological effect. Terms like "active", "active agent", "active pharmaceutical ingredient", "active substance", "drug substance", "active drug" may be used synonymously for "active ingredient". In a preferred embodiment, the edoxaban, or a salt thereof or a hydrate of said edoxaban or edoxaban salt, is edoxaban tosylate monohydrate. Hydrates of edoxaban, and hydrates of edoxaban salts, and therefore explicitly included.

The term "effective amount" or "therapeutically effective amount" used interchangeably, is defined to mean the amount or quantity of the active drug (e.g. edoxaban), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The excipients are described herein in some embodiments according to "wt%", or "percentage by weight". The %wt values recite herein preferably relate to the percentage of material by weight present in the tablet, or in the powder blend prior to compression.

According to the invention, a filler may be used, i.e. as a bulking agent. Various useful fillers include but are not limited to starch, powdered cellulose, microcrystalline cellulose (MCC), calcium phosphate, sugars, such as mannitol, lactose, sorbitol, xylitol, and the like, and mixtures thereof; more preferably microcrystalline cellulose. According to the invention, the preferred filler is lactose, which has been shown to exhibit desired properties with respect to the hardness of the tablet, disintegration properties, stability of the API and dissolution under standard conditions.

Lactose is a disaccharide composed of one galactose and one glucose molecule. In the pharmaceutical industry lactose is used as a filler due to its excellent compressibility properties. Lactose may be used as lactose hydrous, lactose anhydrous, lactose monohydrate, or lactose spray-dried, preferably as lactose monohydrate. In preferred embodiments, the tablets do not contain enough lactose to cause lactose intolerance.

Pregelatinized starch represents a preferred additional filler used preferably in combination with lactose.

Pregelatinized starch (PGS) is generally considered as a filler (diluent), but may also have some disintegrant-like properties, therefore could be considered as "filler-disintegrant". PGS may also have binder-like properties. PGS is therefore a multi-functional excipient. PGS is a starch that has been chemically and/or mechanically processed to rupture all or part of the starch granules. This typically renders the starch flowable and directly compressible (Handbook of Pharmaceutical Excipients (Ed: Rowe)). Partially pregelatinized starch is commercially available. In some embodiments, pregelatinized starch contains 5% of free amylose, 15% of free amylopectin, and 80% unmodified starch. Pregelatinized starch is typically obtained from maize (corn), potato, or rice starch. Pregelatinized starch may be employed in granule or tablet formulations and shows multiple functions as a filler, disintegrant and/or binder,

In the context of the present invention, PGS is defined as a filler.

In the context of the present invention, also microcrystalline cellulose (MCC) is defined only as a filler.

According to the invention, binders hold the ingredients in a tablet together and are preferably employed intragranularly during wet granulation. Binders are the agents used to increase the cohesion of the powdery particles or granules during the compression, in order to obtain pharmaceutical forms with a defined hardness, or to act as processing aid during the granulation process. The binder may be present in the pharmaceutical composition in the form of a single constituent / ingredient or in the form of a mixture of constituents / ingredients. Binders ensure that tablets and granules can be formed with required mechanical strength. The preferred binder is hydroxypropylcellulose. Alternative binders relate to natural binders, semisynthetic polymeric binders, or synthetic polymeric binders.

In the context of the present invention the binder agents are classified into 3 classes: 1) "natural binder", 2) "semisynthetic polymeric binder", and 3) "synthetic polymeric binder".

Due to their natural source the natural binder agents generally exhibit an inherent variability relating to the natural polymers of different batches, which sometimes gives rise to problems in production. To reduce potential processing problems, (natural) binder agents may also be further characterized, e.g., by their viscous properties. In comparison to the natural binder agents, the variability between batches of the semisynthetic and/or synthetic binder agents from the same supplier is reduced due to the adjustment by chemical derivatization or a total chemical synthesis. For almost each polymeric binder agent type, several viscosity grades are generally available.

In the context of the present invention the expression "natural binder" refers to a natural polymer binder or a salt thereof, preferably selected from the group consisting of starch, processed starch or starch salt, e.g., corn starch, potato starch, sodium starch; alginic acid or salts thereof, e.g. sodium alginate; gelatin; Guar gum; gum Arabic; Candelilla wax; Carnauba wax.

In the context of the present invention the expression "semisynthetic polymeric binder" refers to a chemical derivative of natural polymer binder, usually a chemical derivative of cellulose or starch, preferably selected from the group consisting of hydroxypropyl cellulose (HPC, hyprollose), hydroxypropyl methyl cellulose (HPMC, hypromellose), sodium carboxymethyl cellulose, and hydroxypropyl starch.

In the context of the present invention the expression "synthetic polymeric binder" refers to a fully chemically synthesized, non-natural polymer or co-polymer binder agent, preferably selected from the group consisting of povidone, copovidone, polyvinyl alcohol, polyethylene glycol (PEG), polyvinyl caprolactam-polyvinyl acetate-polyethylene glycol graft copolymer, and polyethylene glycol-polyvinyl alcohol graft copolymer (PEG-PVA).

A lubricant, which is an ingredient added to facilitate the tableting process, in particular with regard to compressing granules, is an excipient for the preparation of the tablets as described herein. Lubricants may facilitate flowability of a granule and/or powder to easily fill in a die, may reduce friction between granules and powder themselves and friction among a die, punch, granules and powder, and may facilitate tablet compressing and discharge from a die. In preferred embodiments the lubricant is magnesium stearate. Alternative lubricants relate to talc, glyceryl stearate(s), calcium stearate, sodium stearyl fumarate or stearic acid.

According to the present invention, a disintegrant is typically an agent used in the preparation of solid pharmaceutical formulations which causes them to disintegrate and release their medicinal substances on contact with moisture. In the context of the present invention the expression "disintegrant" refers preferably to the so called class of "superdisintegrants", and preferably does not refer to the class of "filler-disintegrants", such as PGS, These newer substances "superdisintegrants" are more effective at much lower concentrations with greater disintegrating efficiency and mechanical strength compared with "filler-disintgerants", such as starch or PGS. On contact with water, the superdisintegrants swell, hydrate, change volume or form and produce a disruptive change in the tablet. Effective superdisintegrants provide improved compressibility, compatibility and have no negative impact on the mechanical strength of formulations containing high-dose drugs. Superdisintegrants offer significant improvements over "filler-disintgerants", such as PGS.

Disintegrants include but are not limited to hydroxypropyl cellulose (L-HPC), crospovidone, croscarmellose sodium, sodium starch glycolate ans similar other superdisintegrants. According to the invention, the preferred disintegrants are crospovidone and sodium starch glycolate, which have been shown in combination to exhibit desired properties with respect to the hardness of the tablet, disintegration properties, stability of the API and dissolution under standard conditions.

Crospovidone (also known as cross-linked polyvinyl N-pyrrolidone, polyvinyl polypyrrolidone or PVPP) is an inert and insoluble white to light yellow free-flowing powder. It has hygroscopic or water-attracting properties with excellent swelling characteristics. It is this swelling characteristic that makes it useful as a disintegrant in pharmaceutical dosage forms. Crospovidone is not absorbed orally. Oral use of crospovidone is not usually associated with toxicity in normal use as a pharmaceutical excipient.

Sodium starch glycolate is the sodium salt of starch carboxymethyl ether. Starch glycolates are of rice, potato, wheat or corn origin. Sodium starch glycolate is a white to off-white, tasteless, odorless, relatively free flowing powder. Sodium starch glycolate absorbs water rapidly, resulting in swelling which leads to rapid disintegration of tablets and granules. It is used primarily as a disintegrant. According to Handbook of Pharmaceutical Excipients (Ed: Rowe), sodium starch glycolate is widely used in oral pharmaceuticals as a disintegrant, and the usual concentration employed in a formulation is between 2% and 8%, with the optimum concentration about 4%, although in many cases 2% is sufficient.

The composition may also comprise other excipients, such as surfactants, glidants, pigments, and/or antioxidants, as may be desired.

The most commonly used pharmaceutical solid dosage forms today include granules, pellets, tablets and capsules. Tablets are solid pharmaceutical dosage forms containing drug substances with one or more excipients prepared by either compression or molding methods. The basic art of tableting by three well known methods includes direct compression, wet granulation and dry granulation. The present invention preferably encompasses methods of wet granulation and subsequent production of a tablet.

According to the present invention, wet granulation is the process of binding different powder particles together using an adhesive and/or liquid solution. Wet granulation techniques typically involve a granulation liquid, which is preferably a volatile solvent that is easy to remove by drying, which should be non-toxic. The choice of liquid depends on the API and other excipients and can be elected as is required by a skilled person. Examples for wet granulation liquids relate to, for instance, water, ethanol, isopropanol or any other aqueous solution. Various granulation technologies in batch and continuous modes may be employed, such as, without limitation, high shear granulators, fluid bed granulators, twin screw granulators, foam granulators and steam granulators. The process of wet granulation is known to a skilled person and can be adjusted depending on the characteristics of the powders and the available equipment. In the typical wet granulation method, the wet mass is forced through a sieve to produce wet granules that are subsequently dried. A subsequent screening stage breaks agglomerates of granules. Granules are then compressed into tablets.

Preferred embodiments relate to fluid bed granulation. Drug and excipients are loaded into a fluid bed processor, fluidized with air, and granulating fluid is sprayed into the bed, usually from above, with a continuous stream of warm drying air. This often occurs in a three-stage process of blending, in which the drug and excipients are blended with a low volume of fluidizing air to achieve homogeneity and to warm the dry powders. Effective fluidization depends in part on the particle size of the powders to be fluidized, and in practice an easily fluidized powder will have a mean particle size of 30 to 120 µm and many diluents such as milled lactose lie in this range. Granulation follows, in which water or a binder solution is sprayed onto the fluidized bed. Granule growth during this phase depends on a number of factors such as granulating fluid viscosity and droplet size and spray rate. This step is followed by drying, in which the spraying is stopped, and the powder bed is gently fluidized until the granulation is dry. The end point is usually determined by the bed temperature. Advantages of fluidized bed granulation are that it is a contained process, that a single piece of equipment may be used for granulation and drying, thus representing a capital saving over high shear granulation, and that fluid bed processed granules are typically low density and compressible.

After the preparation of granules, they are compressed to obtain a tablet. Tablet compression is typically carried out by either by single punch machine (stamping press) or by multi station machine (rotary press). The tablet press is often a high-speed mechanical device that squeezes the ingredients into the required tablet shape with extreme precision. It can make the tablet in any given shape, although tablets are usually round or oval.

As disclosed in the review article "Superdisintegrant: An overview" by Mohanachandran et al. (International Journal of Pharmaceutical Sciences Review and Research, Volume 6, Issue 1, January - February 2011; Article-022), a disintegrant used in granulated formulation processes can be more effective if used both "intragranularly" and "extragranularly" thereby acting to break the tablet up into granules and having the granules further disintegrate to release the drug substance into solution. However, the portion of disintegrant added intragranularly (in wet granulation processes) is usually not as effective as that added extragranularly due to the fact that it is exposed to wetting and drying (as part of the granulation process) which reduces the activity of the disintegrant. Since a compaction process does not involve its exposure to wetting and drying, the disintegrant used intragranularly tends to retain good disintegration activity. There are three methods of incorporating disintegrating agents into the tablet: A. Internal Addition (Intragranular), B. External Addition (Extragranular), C. Partly Internal and External.

The terms "intragranular" or "intragranular phase" refer to the components within granules or a granulate of the pharmaceutical composition, i.e. those used in preparing a mixture that is granulated, whereas the term "extragranular" relates to additives or excipients added to the granulate, i.e. added to the intragranular phase after generation of initial granules.

Tablet coating is a process by which an essentially dry, outer layer of coating material is applied to the surface of a dosage form. Coating can obtain specific benefits over uncoated tablets. Typically tablet coating involves the application of a sugar or polymeric coat on the tablet. Materials for coating are known to a skilled person and comprise, without limitation, hydroxypropyl methyl cellulose (HPMC, hypromellose) and/or polyethylene glycol (PEG, Macrogol). The advantages of tablet coating may relate to one or more of taste masking, odor masking, physical and chemical protection or control of release profile (used only for modified release products).

For immediate-release tablets, the non-functional coating is applied, which does not change the dissolution profile of core tablets significantly. The non-functional coating is usually applied for the better visual differentiation by color of different tablet strengths in order to avoid medication errors, and for the improving the patient acceptance, e.g. easier swallowing. As disclosed in the US FDA guidance "Size, Shape, and Other Physical Attributes of Generic Tablets and Capsules" (dated June 18, 2015), the presence of a coating can potentially affect the ease of swallowing tablets. The lack of a film coating can also decrease or prevent tablet mobility and increase esophageal transit times compared with a coated tablet of the same size and shape. Coating also can affect other factors that contribute to patient acceptance, such as palatability and smell.

The term "particle size distribution" as used herein refers to the statistical distribution of the volume share related of all particle sizes. Preferred embodiments of the invention use an active ingredient with a particle size distribution (D₉₀) by volume of less than or equal to 40 µm. Within this application, the D90 values for API (without excipients) are determined by the light scattering method, using a Mastersizer 2000 apparatus made by Malvern Instruments. A wet measurement on a dispersion of the particles in a dispersing agent at 25° C is preferred. The D90 value of the integral volume distribution is defined in the context of this invention as the particle diameter, at which 90 percent by volume of the particles have a smaller diameter than the diameter, which corresponds to the D90 value. The particle size distribution according to the invention can be monomodal or bimodal. In the preferred embodiment of the invention the particle size distribution of the active agent is monomodal. The term "monomodal" as used herein refers to only one peak observed in the histogram and/or a graph of the frequency distribution.

The particle size of API in the tablet or capsule can be determined by mapping analysis of the surface of the tablet cut, e.g. using the well-known Raman mapping method.

The edoxaban tablets of the invention are intended for the treatment of medical conditions associated with unwanted blood clots. According to the invention, a blood clot is a gel-like mass formed by platelets and fibrin in the blood to stop bleeding. A blood clot typically forms under healthy conditions to try to repair damage to a blood vessel, either an artery or vein. When blood clots form inappropriately inside an artery or vein, they may cause significant problems because blood flow past the clot is decreased. Deep vein thrombosis (DVT) a medical condition comprising clot formation in a major vein. This can occur in the arms, pelvis, lungs, or brain of a subject. The Centers for Disease Control and Prevention (CDC) of US FDA estimates that DVT, together with pulmonary embolism (a type of venous clot affecting the lungs) affects up to 900,000 Americans each year. These types of blood clots kill approximately 100,000 Americans annually. Additional conditions, that can be caused by and/or are related to unwanted blood clots, are, without limitation, stroke, heart attack, kidney failure, pulmonary embolism, deep vein thrombosis, venous thromboembolism, peripheral artery disease or pregnancy-related problems.

### EXAMPLES

The invention is demonstrated by way of the examples disclosed herein. The examples provide technical support for and a more detailed description of potentially preferred, nonlimiting embodiments of the invention.

### Part A

### Methods for the analysis of the pharmaceutical compositions:

### Tablet Hardness Study:

Hardness of core or film-coated tablets was determined according to the US Pharmacopeia (USP) test.

### Disintegration Study:

Disintegration time of core or film-coated tablets was determined according to the USP test in 37°C deionized water.

### Dissolution Study:

The dissolution tests for the film-coated tablets were carried out according to the dissolution test methods and conditions described in Part **F.**

### Methods for the preparation of the pharmaceutical compositions:

### General procedure for the preparation of compositions below employing a direct compression process:

*Step-1 (Sifting):* Co-Sift the excipients of Stage-A through suitable mesh.
*Step-2 (Dry mixing):* Load the materials from Step-1 in blender and mix for sufficient time.
*Step-3 (Sifting):* Magnesium stearate was sifted through suitable mesh.
*Step-4 (Lubrication and Blending):* Sifted magnesium stearate was added to the blend from Step-2 and blended for sufficient time.
*Step-5 (Compression):* Lubricated blend from Step-4 was compressed into core tablets by compression machine using suitable machine parameters and suitable tooling
*Step-6:* Coating dispersion was prepared by dispersing the corresponding Opadry mix in purified water.
*Step-9 (Coating):* Core tablets from Step-5 were coated with coating dispersion from Step-6 by using coating machine with suitable machine parameters until the needed weight gain is achieved, dried for 10 mins, and then cooled down to room temperature. The film-coated tablets were packed into blisters.

### General procedure for the preparation of compositions below employing a wet granulation process:

*Step-1 (Sifting):* Co-Sift the intragranular excipients of Stage-A through suitable mesh and load in the fluid bed granulator, e.g. Glatt GPCG 1.1 Fluid Bed Dryer.
*Step-2:* Prepare the binder solution by dispersing hydroxypropylcellulose in purified water.
*Step-3 (Granulation):* The blend from Step-1 is granulated by using the binder solution from Step-2. After granulation, the granules are dried in a suitable equipment to get required loss on drying. The dried granules were sifted using suitable mesh.
*Step-4 (Sifting):* Extragranular materials of Stage-C were sifted through suitable mesh.
*Step-5 (Prelubrication):* Sifted extragranular materials of Stage-C (except magnesium stearate) were added to the dried granules from Step-3 and blended for 5 mins.
*Step-6 (Lubrication and Blending):* Sifted magnesium stearate was added to the blend from Step-5 and blended for 5 minutes.
*Step-7 (Compression):* Lubricated blend from Step-6 was compressed into core tablets by compression machine using suitable machine parameters and suitable tooling
*Step-8:* Coating dispersion was prepared by dispersing the corresponding Opadry mix in purified water.
*Step-9 (Coating):* Core tablets from Step-7 were coated with coating dispersion from Step-8 by using coating machine with suitable machine parameters until the needed weight gain is achieved, dried for 10 mins, and then cooled down to room temperature. The film-coated tablets were packed into blisters.

### Part B

**Reference product characterization**

| **Parameter** | **Observation** | | |
|---|---|---|---|
| Brand Name | Lixiana® Filmtabletten | | |
| Generic Name | Edoxaban Film-Coated Tablets | | |
| Strength | 15 mg | 30 mg | 60 mg |
| Description | Orange, round-shaped film-coated tablets (6.7 mm diameter) debossed with "DSC L15" | Pink, round-shaped film-coated tablets (8.5 mm diameter) debossed with "DSC L30" | Yellow, round-shaped film-coated tablets (10.5 mm diameter) debossed with "DSC L60" |
| Label Claim | Each film-coated tablet contains 15 mg / 30 mg / 60 mg Edoxaban | | |
| Conversion | 20.2 mg of Edoxaban tosylate monohydrate is equivalent to 15 mg of Edoxaban | 40.4 mg of Edoxaban tosylate monohydrate is equivalent to 30 mg of Edoxaban | 80.8 mg of Edoxaban tosylate monohydrate is equivalent to 60 mg of Edoxaban |
| Manufactured by | Daiichi Sankyo Europe GmbH, Germany | | |
| Storage | This medicinal product does not require any special storage conditions. | | |
| Lot / Batch No. | 275494 | 283256 | 284144 |
| Expiry Date | 07/2021 | 06/2022 | 07/2022 |
| Average Weight (mg) | 108.1 (107-109) | 214.8 (212-218) | 431.1 (426-437) |
| Thickness (mm) | 3.71 - 3.75 | 4.40 - 4.47 | 5.40 - 5.45 |
| Diameter (mm) | 6.84 - 6.87 | 8.69 - 8.72 | 10.77 - 10.79 |
| Hardness | 53 - 57 | 88 - 89 | 140 - 152 |
| Disintegration Time (min' sec") | 2'21" - 2'32" | 2'40" - 3'24" | 4'34" - 5'10" |
| Pack details | Primary pack: clear PVC/Aluminium blisters | | |

### Part C

### Comparative examples

**Example EDO/C-1 of Edoxaban with mannitol (process: wet granulation):**

| **S. No.** | **Ingredients** | **Mg/tab** |
|---|---|---|
| **Stage-A (Dry mix)** | | |
| 1 | Edoxaban Tosylate Monohydrate *In-House (16 microns)* | 83.230 |
| 2 | Mannitol *Ph. Eur* (Pearlitol 25C) | 195.770 |
| 3 | Lactose Monohydrate *Ph Eur* (Granulac 200) | - |
| 4 | Pregelatinized starch *Ph. Eur* (Starch 1500) | 84.000 |

| **Stage-B (Binder solution)** | | |
|---|---|---|
| 1 | Hydroxypropylcellulose *Ph. Eur* (Klucel ELF) | 12.000 |
| 2 | Water, Purified *Ph. Eur* | q.s. |

| **Stage-B (Blending & Lubrication)** | | |
|---|---|---|
| 1 | Crospovidone *Ph. Eur* (Polyplasdone XL) | 22.000 |
| 2 | Magnesium Stearate *Ph. Eur* | 3.000 |
| | *Total weight of core Tablet* | 400.000 |

| **Stage-C: (Coating)** | | |
|---|---|---|
| 1 | Opadry 03F520440 Yellow | 20.000 |
| 2 | Water, Purified *Ph. Eur* | q.s. |
| | ***Total weight of coated Tablet*** | **420.000** |

**Physical parameters of core and coated tablets:**

| **S. No.** | **Physical Parameters** | **EDO/C-1** |
|---|---|---|
| | **Physical Parameters (Core Tablets)** | |
| 1 | Average Weight (mg) | 399.500 |
| 2 | Thickness (mm) | 5.03 - 5.08 |
| 3 | Hardness (N) | 90 - 101 |
| 4 | Disintegration Time | 7' 50" - 8' 30" |
| 5 | Friability (%) | Nil |

| | **Physical Parameters (Coated Tablets)** | |
|---|---|---|
| 1 | Average Weight (mg) | 419.7 |
| 2 | Thickness (mm) | 5.18 - 5.25 |
| 3 | Hardness (N) | 128 - 144 |
| 4 | Disintegration Time | 10'20" - 11' 00" |

**Example EDO/C-2 of Edoxaban 60 mg film-coated tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 16 µm (Malvern) by volume)* | API | 83.230* |
| 2 | Lactose Monohydrate *Ph.Eur.* (Granulac® 200) | Water-soluble Filler | 195.770 |
| 3 | Pregelatinized Starch (Starch 1500®) | Filler-Disintegrant | 84.000 |

| **Stage-B (Binder solution):** | | | |
|---|---|---|---|
| 1 | Hydroxypropylcellulose *Ph. Eur.* (Klucel® ELF) | Binder | 12.000 |

| 2 | Water, Purified *Ph. Eur.* | Solvent for binder solution | q.s. |
|---|---|---|---|
| **Stage-C (Blending & Lubrication):** | | | |
| 1 | Crospovidone *Ph. Eur.* (Polyplasdone® XL-10) | Superdisintegrant | 22.000 |
| 2 | Magnesium Stearate *Ph. Eur.* | Lubricant | 3.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated tablets:**

| **S. No.** | **Physical Parameters** | **Lixiana® Filmtabletten (Lot No. 284144)** | **EDO/C-2** |
|---|---|---|---|
| **Physical Parameters (Core Tablets)** | | | |
| 1 | Average Weight (mg) | - | 401.0 |
| 2 | Thickness (mm) | - | 5.23 - 5.26 |
| 3 | Hardness (N) | - | 60 - 70 |
| 4 | Disintegration Time (min' sec") | - | 9' 40" - 10' 18" |

| **Physical Parameters (Coated Tablets)** | | | |
|---|---|---|---|
| 1 | Average Weight (mg) | 431.1 | 419.0 |
| 2 | Thickness (mm) | 5.40 - 5.45 | 5.40 - 5.57 |
| 3 | Hardness (N) | 140 - 152 | 91 - 97 |
| 4 | Disintegration Time (min' sec") | 4' 34" - 5' 10" | 10' 30" - 13' 30" |

| | | | |
|---|---|---|---|
| **Note:** The disintegration time (DT) of test product is very high, and not comparable to the DT of the reference product. | | | |

**Example EDO/C-3 of Edoxaban 60 mg film-coated tablets (process: direct compression):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 30 µm (Malvern) by volume)* | API | 83.230* |
| 2 | Lactose Monohydrate *Ph.Eur.* (Tablettose® 100) | Water-soluble Filler | 195.970 |
| 3 | Pregelatinized Starch (Starch 1500®) | Filler-Disintegrant | 84.000 |
| 4 | Crospovidone *Ph. Eur.* (Polyplasdone® XL-10) | Superdisintegrant | 21.400 |
| 5 | Hydroxypropylcellulose *Ph. Eur.* (Klucel® ELF) | Binder | 12.200 |

| **Stage-B (Blending & Lubrication):** | | | |
|---|---|---|---|
| 1 | Magnesium Stearate *Ph. Eur.* | Lubricant | 3.200 |
| ***Total weight of Core Tablets*** | | | **400.000** |

| **Stage-C (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated tablets:**

| **S. No.** | **Physical Parameters** | **Lixiana® Filmtabletten (Lot No. 284144)** | **EDO/C-3** |
|---|---|---|---|
| **Physical Parameters (Core Tablets)** | | | |
| 1 | Average Weight (mg) | - | 401.8 |
| 2 | Thickness (mm) | - | 4.60 - 4.66 |
| 3 | Hardness (N) | - | 90 - 98 |
| 4 | Disintegration Time (min' sec") | - | 0' 35" - 0' 48" |

| **Physical Parameters (Coated Tablets)** | | | |
|---|---|---|---|
| 1 | Average Weight (mg) | 431.1 | 421.6 |
| 2 | Individual Weight (mg) | 426 - 437 | 419 - 426 |
| 3 | Thickness (mm) | 5.40 - 5.45 | 4.80 - 4.84 |
| 4 | Hardness (N) | 140 - 152 | 188 - 202 |
| 5 | Disintegration Time (min' sec") | 4' 34" - 5' 10" | 2' 35" - 3' 03" |

**Example EDO/C-4 of Edoxaban 60 mg film-coated tablets (process: direct compression):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 30 µm (Malvern) by volume)* | API | 83.230* |
| 2 | Lactose Monohydrate *Ph.Eur.* (Tablettose® 100) | Water-soluble Filler | 239.770 |
| 3 | Pregelatinized Starch (Starch 1500®) | Filler-Disintegrant | 40.000 |
| 4 | Crospovidone *Ph. Eur.* (Polyplasdone® XL-10) | Superdisintegrant | 22.000 |
| 5 | Hydroxypropylcellulose *Ph. Eur.* (Klucel® ELF) | Binder | 12.000 |

| **Stage-B (Blending & Lubrication):** | | | |
|---|---|---|---|
| 1 | Magnesium Stearate *Ph. Eur.* | Lubricant | 3.000 |
| ***Total weight of Core Tablets*** | | | **400.000** |

| **Stage-C (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated tablets:**

| **S. No.** | **Physical Parameters** | **Lixiana® Filmtabletten (Lot No. 284144)** | **EDO/C-4** |
|---|---|---|---|
| **Physical Parameters (Core Tablets)** | | | |
| 1 | Average Weight (mg) | - | 400.1 |
| 2 | Thickness (mm) | - | 4.74 - 7.78 |
| 3 | Hardness (N) | - | 79 - 89 |
| 4 | Disintegration Time (min' sec") | - | 0' 38" - 0' 50" |

| **Physical Parameters (Coated Tablets)** | | | |
|---|---|---|---|
| 1 | Average Weight (mg) | 431.1 | 418.5 |
| 2 | Individual Weight (mg) | 426 - 437 | 415 - 425 |
| 3 | Thickness (mm) | 5.40 - 5.45 | 4.89 - 4.96 |
| 4 | Hardness (N) | 140 - 152 | 140 - 147 |
| 5 | Disintegration Time (min' sec") | 4' 34" - 5' 10" | 1' 43" - 2' 05" |

**Example EDO/C-5 of Edoxaban 60 mg film-coated tablets (process: direct compression):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 10 µm (Malvern) by volume)* | API | 83.230* |
| 2 | Lactose Monohydrate *Ph.Eur.* (Tablettose® 100) | Water-soluble Filler | 239.770 |
| 3 | Pregelatinized Starch (Starch 1500®) | Filler-Disintegrant | 40.000 |
| 4 | Crospovidone *Ph. Eur.* (Polyplasdone® XL-10) | Superdisintegrant | 22.000 |
| 5 | Hydroxypropylcellulose *Ph. Eur.* (Klucel® ELF) | Binder | 12.000 |

| **Stage-B (Blending & Lubrication):** | | | |
|---|---|---|---|
| 1 | Magnesium Stearate *Ph. Eur.* | Lubricant | 3.000 |
| ***Total weight of Core Tablets*** | | | **400.000** |

| **Stage-C (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

### Compression observations:

1) Blend flow is not satisfactory.
2) Punch sticking was observed.

**Physical parameters of core & coated tablets:**

| **S. No.** | **Physical Parameters** | **Lixiana® Filmtabletten (Lot No. 284144)** | **EDO/C-5** |
|---|---|---|---|
| **Physical Parameters (Core Tablets)** | | | |
| 1 | Average Weight (mg) | - | 399.0 |
| 2 | Thickness (mm) | - | 4.65 - 4.77 |
| 3 | Hardness (N) | - | 80 - 90 |
| 4 | Disintegration Time (min' sec") | - | 0' 49" |

| **Physical Parameters (Coated Tablets)** | | | |
|---|---|---|---|
| 1 | Average Weight (mg) | 431.1 | 419.0 |
| 2 | Individual Weight (mg) | 426 - 437 | 408 - 430 |
| 3 | Thickness (mm) | 5.40 - 5.45 | 4.85 - 4.97 |
| 4 | Hardness (N) | 140 - 152 | 142 - 149 |
| 5 | Disintegration Time (min' sec") | 4' 34" - 5' 10" | 01' 52" - 2' 00" |

**Example EDO/C-6 of Edoxaban 60 mg film-coated tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 8 µm (Malvern) by volume)* | API | 83.230* |
| 2 | Lactose Monohydrate *Ph.Eur.* (Granulac® 200) | Water-soluble Filler | 195.770 |

| **Stage-B (Binder solution):** | | | |
|---|---|---|---|
| 1 | Hydroxypropylcellulose *Ph. Eur.* (Klucel® LF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 64.000 |
| 2 | Crospovidone *Ph. Eur.* (Polyplasdone® XL-10) | Superdisintegrant | 40.000 |
| 3 | Magnesium Stearate *Ph. Eur.* | Lubricant | 5.000 |
| ***Total weight of Core Tablets*** | | | **400.000** |

| | | | |
|---|---|---|---|
| * *Assay and water content compensated* | | | |

**Physical parameters of coated tablets:**

| **S. No.** | **Physical Parameters** | **Lixiana® Filmtabletten (Lot No. 284144)** | **EDO/C-6** |
|---|---|---|---|
| 1 | Average Weight (mg) | 431.1 | 420.3 |
| 2 | Thickness (mm) | 5.40 - 5.45 | 5.04 - 5.09 |
| 3 | Hardness (N) | 140 - 152 | 101 - 111 |
| 4 | Disintegration Time (min' sec") | 4' 34" - 5' 10" | 09' 15" - 12' 00" |

| | | | |
|---|---|---|---|
| **Note:** The disintegration time (DT) of test product is very high, and not comparable to the DT of the reference product. | | | |

**Example EDO/C-7 of Edoxaban 60 mg film-coated tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 145.770 |

| **Stage-B (Binder solution):** | | | |
|---|---|---|---|
| 1 | Hydroxypropylcellulose *Ph. Eur.* (Klucel® LF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Lactose Monohydrate *Ph.Eur.* (Granulac® 200) | Water-soluble Filler | 30.000 |
| 2 | Pregelatinized Starch (Starch 1500®) | Filler-Disintegrant | 84.000 |
| 3 | Crospovidone *Ph. Eur.* (Polyplasdone® XL-10) | Superdisintegrant | 40.000 |
| 4 | Magnesium Stearate *Ph. Eur.* | Lubricant | 5.000 |
| ***Total weight of Core Tablets*** | | | **400.000** |

| | | | |
|---|---|---|---|
| * *Assay and water content compensated* | | | |

### Process observations:

During the initial phase of granulation, the API was sticking to the walls of the GPCG.

**Physical parameters of core & coated tablets:**

| **S. No.** | **Physical Parameters** | **Lixiana® Filmtabletten (Lot No. 284144)** | **EDO/C-7** |
|---|---|---|---|
| **Physical Parameters (Core Tablets)** | | | |
| 1 | Average Weight (mg) | - | 403.2 |
| 2 | Thickness (mm) | - | 4.86 - 4.91 |
| 3 | Hardness (N) | - | 95 |
| 4 | Disintegration Time (min' sec") | - | 4' 40" - 4' 55" |

| **Physical Parameters (Coated Tablets)** | | | |
|---|---|---|---|
| 1 | Average Weight (mg) | 431.1 | 420.4 |
| 2 | Individual Weight (mg) | 426 - 437 | 417 - 424 |
| 3 | Thickness (mm) | 5.40 - 5.45 | 5.07 - 5.05 |
| 4 | Hardness (N) | 140 - 152 | 105 - 116 |
| 5 | Disintegration Time (min' sec") | 4' 34" - 5' 10" | 6' 40" - 7' 45" |

**Example EDO/C-8 of Edoxaban 60 mg core tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 179.770 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 80.000 |
| 2 | Sodium Starch Glycolate (Explotab® CLV) | Superdisintegrant | 40.000 |
| 3 | Magnesium Stearate *Ph. Eur.* | Lubricant | 5.000 |
| ***Total weight of Core Tablets*** | | | **400.000** |

| | | | |
|---|---|---|---|
| * *Assay and water content compensated* | | | |

**Physical parameters of core tablets:**

| **S. No.** | **Physical Parameters** | **EDO/C-8** |
|---|---|---|
| 1 | Average Weight (mg) | 400.0 |
| 2 | Thickness (mm) | 4.80 - 4.88 |
| 3 | Hardness (N) | 50 - 55 |
| 4 | Disintegration Time (min' sec") | 5' 30" - 6' 00" |

| | | |
|---|---|---|
| ***Note:*** The needed hardness is not achieved with combination of PGS with SSG as disintegrant. | | |

**Example EDO/C-9 of Edoxaban 60 mg core tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 179.770 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Sodium Starch Glycolate (Explotab® CLV) | Superdisintegrant | 80.000 |
| 2 | Magnesium Stearate *Ph. Eur.* | Lubricant | 5.000 |
| ***Total weight of Core Tablets*** | | | **360.000** |

| | | | |
|---|---|---|---|
| * *Assay and water content compensated* | | | |

**Physical parameters of core tablets:**

| **S. No.** | **Physical Parameters** | **EDO/C-9** |
|---|---|---|
| 1 | Average Weight (mg) | 360.0 |
| 2 | Hardness (N) | 35 - 50 |
| 3 | Disintegration Time (min' sec") | 3' 00" |

| | | |
|---|---|---|
| ***Note:*** The needed hardness is not achieved with only SSG as disintegrant. | | |

**Example EDO/C-10 of Edoxaban 60 mg core tablets (process: wet granulation):**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 179.770 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Lactose Monohydrate *Ph.Eur.* (Tablettose® 100) | Water-soluble Filler | 40.000 |
| 2 | Sodium Starch Glycolate (Explotab® CLV) | Superdisintegrant | 80.000 |
| 3 | Magnesium Stearate *Ph. Eur.* | Lubricant | 5.000 |
| ***Total weight of Core Tablets*** | | | **400.000** |

| | | | |
|---|---|---|---|
| * *Assay and water content compensated* | | | |

**Physical parameters of core tablets:**

| **S. No.** | **Physical Parameters** | **EDO/C-10** |
|---|---|---|
| 1 | Average Weight (mg) | 400.0 |
| 2 | Hardness (N) | 50 - 55 |
| 3 | Disintegration Time (min' sec") | 5' 10" |

| | | |
|---|---|---|
| ***Note:*** The needed hardness is not achieved with the combination of lactose monohydrate with SSG in the extragranular phase. | | |

### Part D

### Inventive examples:

All examples below are prepared using the wet granulation process.

**Example EDO/E-1 of Edoxaban 60 mg core tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 179.770 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Crospovidone *Ph. Eur.* (Polyplasdone® XL-10) | Superdisintegrant | 80.000 |
| 2 | Sodium Starch Glycolate (Explotab® CLV) | Superdisintegrant | 40.000 |
| 3 | Magnesium Stearate *Ph. Eur.* | Lubricant | 5.000 |
| ***Total weight of Core Tablets*** | | | **400.000** |

| | | | |
|---|---|---|---|
| * *Assay and water content compensated* | | | |

**Physical parameters of core tablets EDO/E-1 vs. core tablets of comparative examples:**

| **S. No.** | **Physical Parameters** | **Comp. Ex. EDO/C-8** | **Comp. Ex. EDO/C-9** | **Comp. Ex. EDO/C-10** | **Inv. Ex. EDO/E-1** |
|---|---|---|---|---|---|
| 1 | Average Weight (mg) | 400 | 360 | 400 | 400 |
| 2 | Hardness (N) | 50 - 55 | 35 - 50 | 50 - 55 | 85 - 95 |
| 3 | Disintegration Time (min' sec") | 5' 30" - 6' 00" | 3' 00" | 5' 10" | 3' 10" |

| | | | | | |
|---|---|---|---|---|---|
| ***Note:*** The needed hardness of core tablets is achieved with combination of crospovidone with SSG in the inventive example **EDO/E-1,** whilst the hardness was not achieved in the comparative examples **EDO/C-8, EDO/C-9,** and **EDO/C-10.** | | | | | |

**Example EDO/E-2 of Edoxaban 60 mg film-coated tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 8 µm (Malvern) by volume)* | API | 83.230* |
| 2 | Lactose Monohydrate *Ph.Eur.* (Granulac® 200) | Water-soluble Filler | 195.770 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Sodium Starch Glycolate (Explotab® CLV) | Superdisintegrant | 84.000 |
| 2 | Crospovidone *Ph. Eur.* (Polyplasdone® XL-10) | Superdisintegrant | 22.000 |
| 3 | Magnesium Stearate *Ph. Eur.* | Lubricant | 3.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated tablets:**

| **S. No.** | **Physical Parameters** | **Lixiana® Filmtabletten (Lot No. 284144)** | **EDO/E-2** |
|---|---|---|---|
| **Physical Parameters (Core Tablets)** | | | |
| 1 | Average Weight (mg) | - | 400.0 |
| 2 | Thickness (mm) | - | 4.73 - 4.80 |
| 3 | Hardness (N) | - | 80-90 |
| 4 | Disintegration Time (min' sec") | - | 1' 52" |

| **Physical Parameters (Coated Tablets)** | | | |
|---|---|---|---|
| 1 | Average Weight (mg) | 431.1 | 419.7 |
| 2 | Individual weight (mg) | 426 - 437 | 417 - 423 |
| 3 | Thickness (mm) | 5.40 - 5.45 | 4.91 - 4.97 |
| 4 | Hardness (N) | 140 - 152 | 102 - 120 |
| 5 | Disintegration Time (min' sec") | 4' 34" - 5' 10" | 3' 05" - 3' 13" |

**Example EDO/E-3 of Edoxaban 60 mg film-coated tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (1^{st} API source, D₉₀ 7.8 µm (Malvern) by volume)* | API | 83.230* |
| 2 | Lactose Monohydrate *Ph.Eur.* (Granulac® 200) | Water-soluble Filler | 194.770 |
| 3 | Crospovidone *Ph. Eur.* (Polyplasdone® XL-10) | Superdisintegrant | 10.000 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 72.000 |
| 2 | Sodium Starch Glycolate (Explotab® CLV) | Superdisintegrant | 24.000 |
| 3 | Magnesium Stearate *Ph. Eur.* | Lubricant | 4.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated 60 mg tablets:**

| **S. No.** | **Physical Parameters** | **Lixiana® 60 mg Filmtabletten (Lot No. 284144)** | **EDO/E-3** |
|---|---|---|---|
| **Physical Parameters (Core Tablets)** | | | |
| 1 | Average Weight (mg) | - | 401.7 |
| 2 | Thickness (mm) | - | 4.80 - 4.86 |
| 3 | Hardness (N) | - | 70 - 80 |
| 4 | Disintegration Time (min' sec") | - | 3' - 3' 50" |

| **Physical Parameters (Coated Tablets)** | | | |
|---|---|---|---|
| 1 | Average Weight (mg) | 431.1 | 421.66 |
| 2 | Individual weight (mg) | 426 - 437 | 420 - 425 |
| 3 | Thickness (mm) | 5.40 - 5.45 | 4.94 - 4.96 |
| 4 | Hardness (N) | 140 - 152 | 120 - 144 |
| 5 | Disintegration Time (min' sec") | 4' 34" - 5' 10" | 6' 50" - 7' 30" |

**Examples EDO/E-4, EDO/E-5, EDO/E-6, EDO/E-7, and EDO/E-8 of Edoxaban 60 mg film-coated tablets:**

| *Five batches are manufactured using same quantitative composition but using API batches with different particle size distribution (PSD).* | | | |
|---|---|---|---|
| **S. No.** | **Ingredients** | **Function** | **mg/tab** |
| **Stage-A (Dry mix):** | | | |
| 1 | Edoxaban Tosylate Monohydrate *In-House (2^{nd} API source, PSD* - *s. table below)* | API | 83.830* |
| 2 | Lactose Monohydrate *Ph.Eur.* (Granulac® 200) | Water-soluble Filler | 194.170 |
| 3 | Crospovidone *Ph. Eur.* (Polyplasdone® XL-10) | Superdisintegrant | 10.000 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 72.000 |
| 2 | Sodium Starch Glycolate (Explotab® CLV) | Superdisintegrant | 24.000 |
| 3 | Magnesium Stearate *Ph. Eur.* | Lubricant | 4.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Table: Physical parameters of API and core & coated tablets:**

| **S. No.** | **Physical Parameters** | **EDO/E-4** | **EDO/E-5** | **EDO/E-6** | **EDO/E-7** | **EDO/E-8** |
|---|---|---|---|---|---|---|
| **Particle size distribution (PSD) of API** | | | | | | |
| 1 | D₉₀ by volume (Malvern) | 9 µm | 6.4 µm | 30.8 µm | 37.6 µm | 7.6 µm |

| **Physical Parameters (Core Tablets)** | | | | | | |
|---|---|---|---|---|---|---|
| 1 | Average Weight (mg) | 401.4 | 400.45 | 401.1 | 400.0 | 401.1 |
| 2 | Thickness (mm) | 4.81 - 4.84 | 4.83 - 4.90 | 4.84 - 4.87 | 4.87 - 4.94 | 4.86 - 4.91 |
| 3 | Hardness (N) | 84-86 | 88 - 93 | 84-93 | 76 - 83 | 79 - 86 |
| 4 | Disintegration Time (min' sec") | 3' 40" - 3' 50" | 3' 30" - 3' 40" | 3' 40" - 3' 55" | 3' 00" - 3' 20" | 3' 10" - 3' 40" |

| **Physical Parameters (Coated Tablets)** | | | | | | |
|---|---|---|---|---|---|---|
| 1 | Average Weight (mg) | 418.9 | 420.2 | 420.0 | 422.6 | 420.2 |
| 2 | Individual weight (mg) | 417 - 421 | 418 - 425 | 418 - 421 | 420 - 425 | 417 - 424 |
| 3 | Thickness (mm) | 5.01 - 5.06 | 5.04 - 5.12 | 4.98 - 5.03 | 5.10 - 5.16 | 5.02 - 5.08 |
| 4 | Hardness (N) | 104 - 119 | 102 - 123 | 113 - 138 | 107 - 127 | 101 - 124 |
| 5 | Disintegration Time (min' sec") | 5' 52" - 6'40" | 5' 40" - 6' 00" | 5' 30" - 6' 40" | 4' 30" - 5' 40" | 5' 12" - 5' 50" |

**Table: Overview of dissolution profiles in SGF Blank pH 2.1, Paddle, 35 RPM, 250 mL along with IVIVC predictions:**

| **Product / Batch No.** *(PSD)** | **Mean Cumulative % Labelled Amount Dissolved after time (in minutes)** | | | | | | | | | | ***Predicted Ratio (%)*** | |
|---|---|---|---|---|---|---|---|---|---|---|---|---|
| | **5** | **10** | **15** | **20** | **30** | **45** | **60** | **90** | **120** | **Inf.** | ***Cₘₐₓ*** | ***AUC_{Inf}*** |
| **Lixiana® 60 mg / B. No. 284144** | 3 | 20 | 33 | 43 | 56 | 67 | 74 | 79 | 82 | 99 | - | - |
| **EDO/E-5** *(6.4 µm)** | 4 | 13 | 23 | 33 | 50 | 69 | 80 | 87 | 90 | 99 | ***103.6*** | ***109.7*** |
| **EDO/E-7** *(37.6 µm)** | 5 | 15 | 28 | 41 | 59 | 71 | 76 | 82 | 83 | 95 | ***106.6*** | ***101.2*** |
| **EDO/E-8** *(7.6 µm)** | 4 | 12 | 23 | 32 | 52 | 73 | 80 | 84 | 87 | 96 | ***109.8*** | ***106.1*** |

| | | | | | | | | | | | | |
|---|---|---|---|---|---|---|---|---|---|---|---|---|
| ** PSD of used API as D₉₀ by volume (Malvern)* | | | | | | | | | | | | |

As can be seen from the table above, the micronization of Edoxaban API does not have any significant influence on the dissolution of the inventive compositions, as evident from the comparison of the dissolution data of inventive examples having same quantitative composition, but different PSD of used API. Similar dissolution profiles and good IVIVC predictions are observed independently of the PSD of API for both fine API grades *(micronized API)* and the coarse API grade (*non-micronized API*).

**Examples of Edoxaban 15, 30, and 60 mg film-coated tablets:**

| *Analogously to the 60 mg example EDO*/*E-8, the examples of 15 mg and 30 mg strengths* ***EDO*/*E-9 and EDO*/*****E-10** are prepared (dose-proportional composition):* | | | | | |
|---|---|---|---|---|---|
| | | **Batch Number →** | **EDO/E-9** | **EDO/E-10** | **EDO/E-8** |
| **Tablet Strength** (based on Edoxaban free base) → | | | **15 mg** | **30 mg** | **60 mg** |
| **S. No.** | **Ingredients↓** | **Function** | **mg/tab** | **mg/tab** | **mg/tab** |
| **Stage-A (Dry mix):** | | | | | |
| 1 | Edoxaban Tosylate Monohydrate* *In-House* | API | 20.96** | 41.92** | 83.830** |
| 2 | Lactose Monohydrate *Ph.Eur.* (Granulac® 200) | Water-soluble Filler | 48.54 | 97.09 | 194.170 |
| 3 | Crospovidone *Ph. Eur.* (Polyplasdone® XL-10) | Superdisintegrant | 2.500 | 5.000 | 10.000 |

| **Stage-B (Binder solution):** | | | | | |
|---|---|---|---|---|---|
| 1 | Hydroxypropylcellulose *Ph. Eur.* (Klucel® ELF) | Binder | 3.000 | 6.000 | 12.000 |
| 2 | Water, Purified *Ph. Eur.* | Solvent for binder solution | q.s. | q.s. | q.s. |

| **Stage-C (Blending & Lubrication):** | | | | | |
|---|---|---|---|---|---|
| 1 | Pregelatinized Starch (Starch 1500®) | Filler-Disintegrant | 18.000 | 36.000 | 72.000 |
| 2 | Sodium Starch Glycolate (Explotab® CLV) | Superdisintegrant | 6.000 | 12.000 | 24.000 |
| 3 | Magnesium Stearate Ph. *Eur.* | Lubricant | 1.000 | 2.000 | 4.000 |
| ***Total weight of Core Tablets*** | | | **100.000** | **200.000** | **400.000** |

| **Stage-D (Film-Coating):** | | | | | |
|---|---|---|---|---|---|
| 1 | Opadry® coating system*** | Coating | 5.000 | 10.000 | 20.000 |
| 2 | Water, Purified *Ph. Eur.* | Solvent for coating dispersion | q.s. | q.s. | q.s. |
| ***Total weight of Coated Tablets*** | | | **105.000** | **210.000** | **420.000** |

| | | | | | |
|---|---|---|---|---|---|
| ** 2^{nd} API source, D₉₀ 7.6 µm (Malvern) by volume.* ** *Assay and water content compensated.* *** *Used Opadry coating system:* 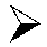 *15 mg film-coated tablets: Opadry*® *03F530101 Orange, comprising hypromellose, talc, macrogol, titanium dioxide, iron oxide yellow, iron oxide red.* 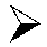 *30 mg film-coated tablets: Opadry*® *03F540319 Pink, comprising hypromellose, talc, macrogol, titanium dioxide, iron oxide red.* 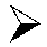 *60 mg film-coated tablets: Opadry*® *03F520440 Yellow, comprising hypromellose, talc, macrogol, titanium dioxide, iron oxide yellow.* | | | | | |

**Examples EDO/E-11, EDO/E-12, and EDO/E-13 of Edoxaban 60 mg core tablets:**

| **Batch Number →** | | | **EDO/E-11** | **EDO/E-12** | **EDO/E-13** |
|---|---|---|---|---|---|
| **S. No.** | **Ingredients↓** | **Function** | **mg/tab** | **mg/tab** | **mg/tab** |
| **Stage-A (Dry mix):** | | | | | |
| 1 | Edoxaban Tosylate Monohydrate* *In-House* | API | 83.830** | 83.830** | 83.830** |
| 2 | Lactose Monohydrate *Ph.Eur.* (Granulac® 200) | Water-soluble Filler | 194.170 | 194.170 | 194.170 |
| 3 | Crospovidone *Ph. Eur.* (Polyplasdone® XL-10) | Superdisintegrant | 10.000 | 10.000 | 10.000 |

| **Stage-B (Binder solution):** | | | | | |
|---|---|---|---|---|---|
| 1 | Hydroxypropylcellulose *Ph. Eur.* (Klucel® ELF) | Binder | 12.000 | 12.000 | 12.000 |
| 2 | Water, Purified *Ph. Eur.* | Solvent for binder solution | q.s. | q.s. | q.s. |

| **Stage-C (Blending & Lubrication):** | | | | | |
|---|---|---|---|---|---|
| 1 | Pregelatinized Starch (Starch 1500®) | Filler-Disintegrant | 62.000 | 48.000 | 56.000 |
| 2 | Sodium Starch Glycolate (Explotab® CLV) | Superdisintegrant | 34.000 | 48.000 | 40.000 |
| 3 | Magnesium Stearate Ph. *Eur.* | Lubricant | 4.000 | 4.000 | 4.000 |
| ***Total weight of Core Tablets*** | | | **400.000** | **400.000** | **400.000** |

| | | | | | |
|---|---|---|---|---|---|
| ** 2^{nd} API source, D₉₀ 7.6 µm (Malvern) by volume.* ** *Assay and water content compensated.* | | | | | |

**Table: Physical parameters of 60 mg core tablets:**

| **S. No.** | **Physical Parameters** | **EDO/E-11** | **EDO/E-12** | **EDO/E-13** |
|---|---|---|---|---|
| 1 | Average Weight (mg) | NT | NT | 401.2 |
| 2 | Thickness (mm) | NT | NT | 4.73 - 4.89 |
| 3 | Hardness (N) | NT | NT | 85 - 92 |
| 4 | Disintegration Time (min' sec") | 3' 20" | 2' 40" | 3' 05" - 3' 20" |

| | | | | |
|---|---|---|---|---|
| *NT: not tested* | | | | |

**Example EDO/E-14 of Edoxaban 60 mg film-coated tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (2^{nd} API source, D₉₀ 7.6 µm (Malvern) by volume)* | API | 83.830* |
| 2 | Lactose Monohydrate *Ph.Eur.* (Granulac® 200) | Water-soluble Filler | 224.170 |
| 3 | Crospovidone *Ph. Eur.* (Polyplasdone® XL-10) | Superdisintegrant | 10.000 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 42.000 |
| 2 | Sodium Starch Glycolate (Explotab® CLV) | Superdisintegrant | 24.000 |
| 3 | Magnesium Stearate *Ph. Eur.* | Lubricant | 4.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * Assay *and water content compensated* | | | |

**Physical parameters of core & coated 60 mg tablets:**

| **S. No.** | **Physical Parameters** | **Lixiana® 60 mg Filmtabletten (Lot No. 284144)** | **EDO/E-14** |
|---|---|---|---|
| **Physical Parameters (Core Tablets)** | | | |
| 1 | Average Weight (mg) | - | 401.3 |
| 2 | Hardness (N) | - | 79-91 |
| 3 | Disintegration Time (min' sec") | - | 3' 25" |

| **Physical Parameters (Coated Tablets)** | | | |
|---|---|---|---|
| 1 | Average Weight (mg) | 431.1 | 420.5 |
| 2 | Individual weight (mg) | 426 - 437 | 418 - 423 |
| 3 | Thickness (mm) | 5.40 - 5.45 | 5.10 - 5.17 |
| 4 | Hardness (N) | 140 - 152 | 123 - 143 |
| 5 | Disintegration Time (min' sec") | 4' 34" - 5' 10" | 5' 20" - 5' 55" |

**Example EDO/E-15 of Edoxaban 60 mg film-coated tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (2^{nd} API source, D₉₀ 7.1 µm (Malvern) by volume)* | API | 83.833* |
| 2 | Lactose Monohydrate *Ph.Eur.* (Granulac® 200) | Water-soluble Filler | 169.167 |
| 3 | Crospovidone *Ph. Eur.* (Polyplasdone® XL-10) | Superdisintegrant | 10.000 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Pregelatinized Starch (Starch 1500®) | Filler-Disintegrant | 72.000 |
| 2 | Sodium Starch Glycolate (Explotab® CLV) | Superdisintegrant | 24.000 |
| 3 | Crospovidone *Ph. Eur.* (Polyplasdone® XL) | Superdisintegrant | 25.000 |
| 4 | Magnesium Stearate *Ph. Eur.* | Lubricant | 4.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated 60 mg tablets:**

| **S. No.** | **Physical Parameters** | **Lixiana® 60 mg Filmtabletten (Lot No. 284144)** | **EDO/E-15** |
|---|---|---|---|
| **Physical Parameters (Core Tablets)** | | | |
| 1 | Average Weight (mg) | - | 400.4 |
| 2 | Thickness (mm) | - | 5.03-5.08 |
| 3 | Hardness (N) | - | 79 - 84 |
| 4 | Disintegration Time (min' sec") | - | 2' 08" - 2' 30" |

| **Physical Parameters (Coated Tablets)** | | | |
|---|---|---|---|
| 1 | Average Weight (mg) | 431.1 | 420.1 |
| 2 | Individual weight (mg) | 426 - 437 | 418 - 424 |
| 3 | Thickness (mm) | 5.40 - 5.45 | 5.27 - 5.32 |
| 4 | Hardness (N) | 140 - 152 | 110 - 125 |
| 5 | Disintegration Time (min' sec") | 4' 34" - 5' 10" | 4' 37" - 4' 43" |

**Example EDO/E-16 of Edoxaban 60 mg film-coated tablets:**

| **S. No.** | **Ingredients** | **Function** | **mg/tab** |
|---|---|---|---|
| **Stage-A (Dry mix):** | | | |
| 1 | Edoxaban Tosylate Monohydrate *In-House (2^{nd} API source, D₉₀ 37.7 µm (Malvern) by volume)* | API | 83.833* |
| 2 | Lactose Monohydrate *Ph.Eur.* (Granulac® 200) | Water-soluble Filler | 194.167 |

| **Stage-B (Binder solution):** | | | |
|---|---|---|---|
| 1 | Hydroxypropylcellulose *Ph. Eur.* (Klucel® ELF) | Binder | 12.000 |
| 2 | Water, Purified *Ph. Eur.* | Solvent for binder solution | q.s. |

| **Stage-C (Blending & Lubrication):** | | | |
|---|---|---|---|
| 1 | Sodium Starch Glycolate (Explotab® CLV) | Superdisintegrant | 84.000 |
| 2 | Crospovidone *Ph. Eur.* (Polyplasdone® XL) | Superdisintegrant | 22.000 |
| 3 | Magnesium Stearate *Ph. Eur.* | Lubricant | 4.000 |
| ***Total weight of Core Tablets*** | | | **400.000** |

| **Stage-D (Film-Coating):** | | | |
|---|---|---|---|
| 1 | Opadry® 03F520440 Yellow | Coating | 20.000 |
| 2 | Water, Purified *Ph. Eur.* | Solvent for coating dispersion | q.s. |
| ***Total weight of Film-Coated Tablets*** | | | **420.000** |

| | | | |
|---|---|---|---|
| * *Assay and water content compensated* | | | |

**Physical parameters of core & coated 60 mg tablets:**

| **S. No.** | **Physical Parameters** | **Lixiana® 60 mg Filmtabletten (Lot No. 284144)** | **EDO/E-16** |
|---|---|---|---|
| **Physical Parameters (Core Tablets)** | | | |
| 1 | Average Weight (mg) | - | 402.7 |
| 2 | Thickness (mm) | - | 4.77 - 4.82 |
| 3 | Hardness (N) | - | 79 - 85 |
| 4 | Disintegration Time (min' sec") | - | 1'46" - 1'52" |

| **Physical Parameters (Coated Tablets)** | | | |
|---|---|---|---|
| 1 | Average Weight (mg) | 431.1 | 419.1 |
| 2 | Individual weight (mg) | 426 - 437 | 416 - 422 |
| 3 | Thickness (mm) | 5.40 - 5.45 | 4.96 - 5.02 |
| 4 | Hardness (N) | 140 - 152 | 94 - 103 |
| 5 | Disintegration Time (min' sec") | 4' 34" - 5' 10" | 2' 27" - 2' 36" |

### Part E

### Dissolution Studies:

For the film-coated tablet formulations of inventive examples and comparative examples, the dissolution test was carried out according to the following dissolution test methods and conditions.

### Dissolution conditions and methods:

The dissolution testing in the quality control (QC) release media at pH 4.5 was carried out using tablets at a paddle speed of 50 revolutions per minute (RPM) according to method 2 (Paddle) of dissolution test of the US Pharmacopeia (USP), using 900 mL of acetate buffer at pH 4.5. The temperature of media is maintained at 37°C ± 0.5°C using a water bath. Sample solutions were obtained at 5, 10, 15, 20, 30, 45 and 60 minutes after starting the test, and filtered through a 0.45 µm PVDF Millipore syringe filter.

The dissolution testing at pH 6.8 was carried out using tablets according to method 2 of dissolution test of the USP, using 1000 mL of Phosphate buffer solution pH 6.8 at a paddle speed of 35 RPM (up to 30 min) and 50 RPM (30 - 120 min). The temperature of media is maintained at 37°C ± 0.5°C using a water bath. Sample solutions were obtained at 5, 10, 15, 20, 30, 45 60, 90, and 120 minutes after starting the test, and filtered through a 0.45 µm PVDF Millipore syringe filter.

The dissolution testing at pH 2.1 was carried out using tablets according to method 2 of dissolution test of the USP, using 250 mL of SGF Blank pH 2.1 at a paddle speed of 35 RPM. The temperature of media is maintained at 37°C ± 0.5°C using a water bath. Sample solutions were obtained at 5, 10, 15, 20, 30, 45 60, 90, and 120 minutes after starting the test, and filtered through a 0.45 µm PVDF Millipore syringe filter.

### Dissolution analytical test method details:

Equipment: A High-Performance Liquid Chromatographic system with gradient/isocratic elution capability, a spectrophotometric UV detector and an auto sampler was employed (Waters Alliance 2695 separations module, Waters 2487 dual λ absorbance detector or equivalent).

### Preparation of mobile phase:

Buffer: Weigh accurately 2.72 g of Potassium dihydrogen phosphate and 3.0 g of 1-Octane sulfonic acid sodium salt into a beaker containing 1000 mL of Milli-Q- grade water and mix to dissolve. Adjust the pH to 5.5 with diluted potassium hydroxide solution and Filter through 0.45µ or finer porosity PVDF membrane filter and degas
Mobile phase: Mix Buffer and Acetonitrile in the ratio of 60:40 v/v and degas.
Diluent: Mix Acetonitrile and Methanol in the ratio 50:50 v/v

### Preparation of solutions:

Standard stock solution: Prepare a solution containing 0.60 mg/mL of Edoxaban in diluent.

For example, weigh accurately 78 mg of Edoxaban tosylate monohydrate working standard into a 100 mL clean, dry volumetric flask. Add 30 mL of diluent and sonicate for few minutes. Make up to volume with diluent and mix. Label this stock solution as the Standard Stock-1. Prepare in duplicate and label it as Standard Stock-2.

Standard solution for media with pH 4.5 (QC Media):
Prepare a solution containing 0.06 mg/mL of Edoxaban in dissolution medium. For example: Dilute 5.0 mL of standard stock-1 solution to 50 mL with dissolution medium and mix. Label this solution as STD-1. Similarly prepare STD-2 using respective standard stock-2 solution.

Standard stock solution for alternate media: Prepare a solution containing 0.75 mg/mL of Edoxaban in diluent.

For example, weigh accurately 50 mg of Edoxaban tosylate monohydrate working standard into a 50 mL clean, dry volumetric flask. Add 30 mL of diluent and sonicate for few minutes. Make up to volume with diluent and mix. Label this stock solution as the Standard Stock-1. Prepare in duplicate and label it as Standard Stock-2.

Standard solution for media with pH 2.1:
Prepare a solution containing 0.06 mg/mL of Edoxaban in dissolution medium. For example: Dilute 4.0 mL of standard stock-1 solution to 50 mL with dissolution medium and mix. Label this solution as STD-1. Similarly prepare STD-2 using respective standard stock-2 solution.

Standard solution for media with pH 6.8:
Prepare a solution containing 0.24 mg/mL of Edoxaban in dissolution medium. For example: Dilute 8.0 mL of standard stock-1 solution to 25 mL with dissolution medium and mix. Label this solution as STD-1. Similarly prepare STD-2 using respective standard stock-2 solution.

### HPLC chromatographic conditions:

Column: Inertsil ODS-3V 250x4.6 mm, 5 µm
Flow rate: 1.5 mL/min
Detection: UV, 210 nm
Injection Volume: 20 µL
Data acquisition time: 8 minutes
Pump mode: Isocratic
Column temperature: 40° C

### Results of preliminary comparison in dissolution media with pH 2.1 and 6.8 (EDO/C-2 vs EDO/C-1):

Tablet dissolution in media with either pH 2.1 or pH 6.8 was tested at a paddle speed of 35 RPM using 250 mL of SGF Blank pH 2.1, or phosphate buffer solution pH 6.8, 1000 ml, paddle speed 35 RPM, respectively.

**Table: Mean Cumulative % Drug Release; SGF (Blank) pH 2.1, 250 ml, 35 rpm**

| | | | | | | | | | | | | |
|---|---|---|---|---|---|---|---|---|---|---|---|---|
| - | Mean Cumulative % Drug Release | | | | | | | | | | | |
| Media | SGF (Blank) pH 2.1, 250 ml, Paddle, 35 rpm' | | | | | | | | | | | |
| Time (Min) | 5 | 10 | 15 | 20 | 30 | 45 | 60 | 90 | 120 | Infinity | Cmax | AUC |
| **Lixiana® (284144)** | 3 | 20 | 33 | 43 | 56 | 67 | 74 | 79 | 82 | 99 | - | - |
| **EDO/C-1** | 1 | 6 | 13 | 19 | 31 | 43 | 56 | 75 | 84 | 98 | 64.8 | 102.4 |
| **EDO/C-2** | 1 | 6 | 12 | 18 | 30 | 47 | 64 | 84 | 95 | 98 | 70.8 | 115.8 |

**Table: Mean Cumulative % Drug Release; Phosphate buffer pH 6.8, 1000 ml, 35 rpm**

| | | | | | | | | | | | | |
|---|---|---|---|---|---|---|---|---|---|---|---|---|
| - | Mean Cumulative % Drug Release | | | | | | | | | | | |
| **Media** | Phosphate buffer solution pH 6.8, 1000 ml, Paddle, 35 rpm (upto 30 min), 50 rpm (30 - 120 min) | | | | | | | | | | | |
| **Time (Min)** | 5 | 10 | 15 | 20 | 30 | 45 | 60 | 90 | 120 | Infinity | Cmax | AUC |
| **Lixiana® (284144)** | 8 | 34 | 48 | 54 | 61 | 69 | 73 | 79 | 80 | 93 | - | - |
| **EDO/C-1** | 0 | 2 | 5 | 12 | 30 | 49 | 52 | 61 | 65 | 81 | 73.0 | 80.6 |
| **EDO/C-2** | 1 | 3 | 6 | 7 | 13 | 23 | 32 | 42 | 50 | 71 | 33.9 | 62.0 |

As can be seen from the tables above, both **EDO/C-1** and **EDO/C-2** diverged from the reference product Lixiana at both pH conditions 2.1 and 6.8. However, under pH 2.1 the compositions **EDO/C-1** and **EDO/C-2** both showed similar dissolution properties, although **EDO/C-2** with lactose was slightly improved over the mannitol composition. Under conditions of pH 6.8 the mannitol containing composition **EDO/C-1** showed faster dissolution compared to **EDO/C-2.**

These results again indicate the variability of dissolution properties when employing edoxaban compositions, such as those of the prior art. These results also vary from the results obtained in WO 2008/129846. However, due to the somewhat improved results for lactose containing compositions, in contrast to the statements in WO 2008/129846, further attempts were carried out in employing lactose in the inventive compositions.

### Results of dissolution tests in QC release media with pH 4.5:

The dissolution in the quality control (QC) release media was tested according to method 2 of USP dissolution test at a paddle speed of 50 RPM using 900 mL of acetate buffer at pH 4.5.

Based on the European Pharmacopoeia (Ph. Eur. 5.17.1) recommendation for immediate-release dosage forms, the specification is set as at least 75% of the active substance is dissolved within 45 minutes in the QC release media.

The experimental data of the table below shows that 75 wt.% or more, preferably more than 80 wt.%, more preferably more than 90 wt.% of Edoxaban of the inventive pharmaceutical tablet compositions is dissolved within 45 minutes in accordance with the regulatory standard test.

**Table: Dissolution in QC release media**

| **Example** | **Cumulative wt.% edoxaban after 45 minutes** |
|---|---|
| **Lixiana® 60 mg (B. No. 284144)** | 100 |
| **EDO/C-2** | 49 |
| **EDO/C-3** | 98 |
| **EDO/E-4** | 98 |
| **EDO/E-5** | 99 |
| **EDO/E-6** | 99 |
| **Lixiana® 30 mg (B. No. 283256)** | 101 |
| **EDO/E-10** | 104 |
| **Lixiana® 15 mg (B. No. 275494)** | 99 |
| **EDO/E-9** | 99 |

As can be seen from the table above, the dissolution of the inventive formulations of the present invention in QC release media (acetate buffer pH 4.5, Paddle-50 RPM, 900 mL) is comparable to the commercially available reference product (Lixiana® film-coated tablets).

In comparison thereto, the comparative composition **EDO/C-2** does not fulfil the regulatory standard test for dissolution, as only 49 wt.% of edoxaban is dissolved within 45 minutes.

### Results of dissolution tests in media with pH 6.8:

As can be seen in the two tables below, the example **EDO/E-8** according to the present invention shows comparable dissolution at the end point to the commercially available reference product (Lixiana® 60 mg) when assessed in pH 6.8 media.

The variation of the dissolution between single tablets of the same batch is very low, as can be seen on the individual values and the calculated value for the relative standard deviation (RSD). The RSD of the inventive example is lower compared with the reference product Lixiana.

**Table: Dissolution of example EDO/E-8 after 120 minutes (end point) in Phosphate buffer solution pH 6.8, Paddle, 35 RPM (up to 30 min), 50 RPM (30 - 120 min), 1000 mL along with RSD values**

| **Batch EDO/E-8, Tablet-No:** | 1 | 2 | 3 | 4 | 5 | 6 |
|---|---|---|---|---|---|---|
| **Cumulative % Labelled amount (dissolved at 120 minutes)** | 85 | 85 | 85 | 84 | 86 | 84 |
| **Mean** | 85 | | | | | |
| **% RSD** | 0.89 | | | | | |

As shown in the table below, the examples according to the present invention show comparable dissolution at the end point (120 minutes) to the commercially available reference product (Lixiana® 60 mg) when assessed in pH 6.8 media. The variation of the dissolution between single tablets of the same batch is very low, as can be seen on the relative standard deviation (RSD) values. The RSD values of the inventive examples are lower compared with the reference product Lixiana and the comparative examples. This indicates clearly a reduced variability in dissolution compared to the prior art.

**Table: Dissolution after 120 minutes (end point) in Phosphate buffer solution pH 6.8, Paddle, 35 RPM (up to 30 min), 50 RPM (30 - 120 min), 1000 mL along with RSD values**

| **Product / Batch No.** | **Mean Cumulative % Labelled Amount Dissolved after 120 minutes** | **RSD (%) at 120 minutes** |
|---|---|---|
| **Reference product:** | | |
| **Lixiana® 60 mg / B. No. 284144** | 80 | **4.6** |

| **Comparative examples:** | | |
|---|---|---|
| **EDO/C-3** | 57 | **1.75** |
| **EDO/C-4** | 52 | **5.09** |
| **EDO/C-5** | 58 | **7.71** |
| **EDO/C-1** | 65 | **2.33** |
| **EDO/C-2** | 50 | **4.38** |
| **EDO/C-7** | 70 | **1.67** |

| **Inventive examples:** | | |
|---|---|---|
| **EDO/E-2** | 86 | **0.88** |
| **EDO/E-3-60mg** | 84 | **0.97** |
| **EDO/E-4** | 85 | **0.74** |
| **EDO/E-7** | 78 | **0.97** |
| **EDO/E-8** | 85 | **0.89** |
| **EDO/E-15** | 86 | **0.64** |
| **EDO/E-16** | 85 | **0.48** |

As shown in the table below, the examples according to the present invention show also comparable complete dissolution profiles to the commercially available reference product (Lixiana® 60 mg) when assessed in pH 6.8 media. The comparability with the reference product is also evident when shown with In-vitro In-vivo correlation (IVIVC) prediction for Cmax and Auc values.

**Table: Overview of dissolution profiles in Phosphate buffer solution pH 6.8, Paddle, 35 RPM (up to 30 min), 50 RPM (30 - 120 min), 1000 mL along with IVIVC predictions**

| **Product / Batch No.** | **Mean Cumulative % Labelled Amount Dissolved after time (in minutes)** | | | | | | | | | | ***Predicted Ratio (%)*** | |
|---|---|---|---|---|---|---|---|---|---|---|---|---|
| | **5** | **10** | **15** | **20** | **30** | **45** | **60** | **90** | **120** | **Inf.** | ***Cₘₐₓ*** | ***AUC_{Inf}*** |
| **Reference product:** | | | | | | | | | | | | |
| **Lixiana® 60 mg / B. No. 284144** | 8 | 34 | 48 | 54 | 61 | 69 | 73 | 79 | 80 | 93 | - | - |

| **Comparative examples:** | | | | | | | | | | | | |
|---|---|---|---|---|---|---|---|---|---|---|---|---|
| **EDO/C-3** | 12 | 20 | 25 | 29 | 34 | 42 | 46 | 53 | 57 | 71 | ***65.9*** | ***70.4*** |
| **EDO/C-4** | 4 | 13 | 14 | 17 | 24 | 33 | 38 | 46 | 52 | 68 | ***48.3*** | ***64.9*** |
| **EDO/C-5** | 7 | 13 | 18 | 23 | 30 | 38 | 45 | 53 | 58 | 82 | ***67.4*** | ***71.8*** |
| **EDO/C-2** | 1 | 3 | 6 | 7 | 13 | 23 | 32 | 42 | 50 | 71 | ***33.9*** | ***62.0*** |
| **EDO/C-7** | 2 | 11 | 25 | 32 | 37 | 47 | 55 | 67 | 70 | 83 | ***68.8*** | ***87.6*** |

| **Inventive examples:** | | | | | | | | | | | | |
|---|---|---|---|---|---|---|---|---|---|---|---|---|
| **EDO/E-2** | 27 | 48 | 55 | 60 | 69 | 76 | 79 | 84 | 86 | 90 | ***108.7*** | ***106.1*** |
| **EDO/E-3-60mg** | 1 | 6 | 17 | 32 | 53 | 69 | 75 | 82 | 84 | 88 | ***102.8*** | ***105.0*** |
| **EDO/E-4** | 2 | 10 | 22 | 37 | 53 | 68 | 75 | 82 | 85 | 90 | ***100.7*** | ***106.2*** |
| **EDO/E-7** | 3 | 12 | 28 | 40 | 51 | 63 | 68 | 74 | 78 | 82 | ***92.8*** | ***97.5*** |
| **EDO/E-8** | 2 | 12 | 27 | 43 | 58 | 69 | 75 | 81 | 85 | 90 | ***102.0*** | ***106.2*** |
| **EDO/E-16** | 17 | 40 | 50 | 57 | 65 | 73 | 77 | 82 | 85 | 88 | ***101.9*** | ***100.β*** |

As such, it can be drawn from the data above that the inventive compositions of the present invention, as defined by the presence of lactose, crospovidone and SSG, show improved solubility over comparative examples **EDO/C-3, EDO/C-4, EDO/C-5, EDO/C-2, and EDO/C-7.**

Dissolution of the inventive formulations of the present invention in media with pH 6.8 is comparable to the commercially available reference product, as is required in order to achieve a bioequivalent product to Lixiana®. The comparability with the reference product is also evident when shown with In-vitro In-vivo correlation (IVIVC) prediction, calculated with WinNonlin software based on dissolution profiles in the biorelevant media. Based on IVIVC calculation for above shown compositions, the predicted Cmax is 92.8-108.7%, and predicted AUC Inf is 97.5-106.2%, being in the regulatory needed interval of 80-125% for Cmax and AUC.

The dissolution profiles for the comparative examples **EDO/C-3, EDO/C-4, EDO/C-5, EDO/C-2, and EDO/C-7** are not comparable to the reference product Lixiana® and to the inventive examples, as the drug release in the media with pH 6.8 is significantly lower at 60 min time point for test product compared with the reference product (42-67% vs. 79%).

The missing comparability of **EDO/C-3, EDO/C-4, EDO/C-5, EDO/C-2,** and **EDO/C-7** with the reference product Lixiana® is also evident from the IVIVC prediction. The predicted Cmax is 33.9-68.8% being significantly below the regulatory needed interval of 80-125% for Cmax. Also the predicted AUC Inf values of 62-71.8% for comparative examples **EDO/C-3, EDO/C-4, EDO/C-5, EDO/C-2** are significantly below the regulatory needed interval of 80-125% for Cmax.

### Results of dissolution tests in media with pH 2.1:

The examples according to the present invention show comparable dissolution profiles to the commercially available reference product (Lixiana® 60 mg) when assessed in SGF Blank pH 2.1 media. As such, it can be drawn from these data that the inventive compositions of the present invention, as defined by the presence of lactose, crospovidone and SSG, show improved solubility over comparative example **EDO/C-6.**

Dissolution of the inventive formulations of the present invention in media with pH 2.1 is comparable to the commercially available reference product, as is required in order to achieve a bioequivalent product to Lixiana®. The comparability with the reference product is also evident when shown with In-vitro In-vivo correlation (IVIVC) prediction, calculated with WinNonlin software based on dissolution profiles in the biorelevant media. Based on IVIVC calculation for above shown compositions, the predicted Cmax is 103.6-109.8%, and predicted AUC Inf is101.2-109.6%, being in the regulatory needed interval of 80-125% for Cmax and AUC.

The dissolution profile for the comparative example **EDO/C-6** is not comparable to the reference product Lixiana® and to the inventive examples, as the drug release in the media with pH 2.1 is significantly lower at 60 min time point for test product compared with the reference product (46% vs. 74%). The missing comparability of **EDO/C-6** with the reference product Lixiana® is also evident from the IVIVC prediction: the predicted Cmax is 55.4% being significantly below the regulatory needed interval of 80-125% for Cmax.

**Table: Overview of dissolution profiles in SGF Blank pH 2.1, Paddle, 35 RPM, 250 mL along with IVIVC predictions:**

| **Product / Batch No.** | **Mean Cumulative % Labelled Amount Dissolved after time (in minutes)** | | | | | | | | | | ***Predicted Ratio (%)*** | |
|---|---|---|---|---|---|---|---|---|---|---|---|---|
| | **5** | **10** | **15** | **20** | **30** | **45** | **60** | **90** | **120** | **Inf.** | ***Cₘₐₓ*** | ***AUC_{Inf}*** |
| **Reference product:** | | | | | | | | | | | | |
| **Lixiana® 60 mg / B. No. 284144** | 3 | 20 | 33 | 43 | 56 | 67 | 74 | 79 | 82 | 99 | **-** | **-** |

| **Comparative examples:** | | | | | | | | | | | | |
|---|---|---|---|---|---|---|---|---|---|---|---|---|
| **EDO/C-6** | 2 | 8 | 13 | 18 | 26 | 37 | 46 | 62 | 73 | 93 | ***55.4*** | ***89.0*** |

| **Inventive examples:** | | | | | | | | | | | | |
|---|---|---|---|---|---|---|---|---|---|---|---|---|
| **EDO/E-3-60mg** | 7 | 15 | 25 | 35 | 52 | 72 | 82 | 88 | 90 | 99 | ***107.9*** | ***109.6*** |
| **EDO/E-5** | 4 | 13 | 23 | 33 | 50 | 69 | 80 | 87 | 90 | 99 | ***103.6*** | ***109.7*** |
| **EDO/E-7** | 5 | 15 | 28 | 41 | 59 | 71 | 76 | 82 | 83 | 95 | ***106.6*** | ***101.2*** |
| **EDO/E-8** | 4 | 12 | 23 | 32 | 52 | 73 | 80 | 84 | 87 | 96 | ***109.8*** | ***106.1*** |

The results above therefore demonstrate that not only do the inventive compositions show improved dissolution properties over the prior art, and similar dissolution to the reference product Lixiana, but these dissolution properties are more consistent at various pH conditions (i.e. at both pH 2.1 and 6.8), thereby representing an improvement over edoxaban compositions disclosed in the art.

### Part G

### Stability Studies:

Inventive Edoxaban 60 mg film-coated tablets were loaded into the below mentioned conditions and tablets were analyzed in the corresponding tests (Water by KF, Dissolution, Related Substance, and Assay) at respective time points.

**Table: Stability data of Edoxaban 60 mg tablets batch EDO/E-5 at ICH Accelerated condition (40°C/75 % RH) in Clear PVC - Alu blister pack:**

| **Test** | **Specification** | | **Initial** | **3 months** |
|---|---|---|---|---|
| **Water (% m/m by Karl-Fischer**) | Not more than 8.0 | | 5.57 | 5.38 |
| **Dissolution (% Labelled amount, by HPLC)** | Not less than 75% (Q) of the labeled amount of Edoxaban is dissolved in 45 minutes. | Mean | 99 | 98 |
| | | Min. | 98 | 97 |
| | | Max. | 99 | 99 |
| **Related Substances (% m/m, by HPLC)** | | | | |
| **a. Unknown Individual Impurity** | Not more than 0.2 | | 0.16 | 0.11 |
| **b. Total impurities** | Not more than 1.0 | | 0.26 | 0.27 |
| **Assay (% m/m, by HPLC)** | 95.0 -105.0 | | 99.6 | 99.3 |

As can be observed from the table above, the dissolution profile of the test formulation **EDO/E-**5 shows comparable dissolution profile after the 3-months storage of the tablets under ICH accelerated conditions (40°C/75% RH) in Clear PVC-Alu blister, when compared to the fresh tablets. No changes are observed (like a drop in the dissolution).

There is also no significant influence of temperature and humidity observed on Related Substances of Edoxaban.

Furthermore, the Assay of tablets demonstrates a value of 99.6% (initial) and 99.3% (after 3 months), correspondingly, clearly satisfying the standard requirements of 95-105%.

## Claims

1. A pharmaceutical composition in the form of a tablet, comprising a) edoxaban, or a salt thereof or a hydrate of said edoxaban or edoxaban salt, as an active ingredient, b) lactose as a water-soluble filler, and c) crospovidone and sodium starch glycolate as disintegrants.

2. The pharmaceutical composition according to any one of the preceding claims, wherein the active ingredient has a particle size distribution (D₉₀ by volume) of less than or equal to 40 µm.

3. The pharmaceutical composition according to any one of the preceding claims, wherein the composition comprises additionally a binder, an additional filler and a lubricant, or wherein the composition comprises additionally a binder and a lubricant without an additional binder.

4. The pharmaceutical composition according to claim 3, wherein the binder is hydroxypropylcellulose, wherein the additional filler is pregelatinized starch, and/or wherein the lubricant is magnesium stearate.

5. The pharmaceutical composition according to any one of the preceding claims, wherein said composition is prepared by wet granulation, preferably fluid bed granulation, of a blend of at least components a) and b) and optionally c) of claim 1 to produce granules, optional addition of one or more components c) to the granules, such that both crospovidone and sodium starch glycolate are present, and compression of the granules to a tablet.

6. The pharmaceutical composition according to any one of the preceding claims, wherein crospovidone and sodium starch glycolate are extragranular (added after wet granulation).

7. The pharmaceutical composition according to any one of the preceding claims, wherein an additional filler and a lubricant are extragranular.

8. The pharmaceutical composition according to any one of the preceding claims, wherein the tablet is coated, preferably wherein the coating is a film coating comprising hydroxypropyl methyl cellulose (HPMC, hypromellose) and/or polyethylene glycol (PEG, Macrogol).

9. The pharmaceutical composition according to any one of the preceding claims, comprising:
a. edoxaban, or a salt thereof or a hydrate of said edoxaban or edoxaban salt, preferably edoxaban tosylate monohydrate, present in an amount of 10-30 wt%, preferably 15-25 wt%, more preferably about 18-22 wt%,
b. lactose, preferably lactose monohydrate, present in an amount of 20-70 wt%, preferably 30-60 wt%, more preferably about 35-55 wt%,
c. crospovidone, present in an amount of 0.5-25 wt%, preferably 1-10 wt%, more preferably about 1.5-8 wt%,
d. sodium starch glycolate, present in an amount of 1-25 wt%, preferably 2-20 wt%, more preferably about 4-15%,
based on the total weight of all components of the tablet.

10. The pharmaceutical composition according to the preceding claim, comprising additionally:
e. lubricant, preferably magnesium stearate, present in an amount of 0.1-5 wt%, preferably 0.25-2.5 wt%, more preferably about 0.5-2 wt%,
f. optionally pregelatinized starch, present in an amount of 0-30 wt%, preferably 4-20 wt%, more preferably about 5-18 wt%,
g. hydroxypropylcellulose, present in an amount of 1-5 wt%, preferably 2-4 wt%, more preferably about 2.5-3.5 wt%,
h. a coating agent, preferably comprising hydroxypropyl methyl cellulose and/or polyethylene glycol (PEG, Macrogol), present in an amount of 1-10 wt%, preferably 3-7 wt%, more preferably about 4-6 wt%,
based on the total weight of all components of the tablet.

11. The pharmaceutical composition according to any one of the preceding claims,
wherein the tablet shows at least 75% dissolution of the active ingredient after 45 minutes using method 2 of the dissolution test of the US Pharmacopeia (USP), with a paddle speed of 50 revolutions per minute in 900 mL of acetate buffer at pH 4.5.

12. The pharmaceutical composition according to any one of the preceding claims for use as a medicament in the treatment and/or prevention of a medical condition associated with unwanted blood clots, such as stroke, systemic embolism, non-valvular atrial fibrillation, venous thromboembolism, deep-vein thrombosis and/or pulmonary embolism.

13. Method of preparing a pharmaceutical composition in the form of a tablet, the method comprising wet granulation, preferably fluid bed granulation, of a blend of at least components a) and b) and optionally c) of claim 1 to produce granules, optional addition of one or more components c) to the granules, such that both crospovidone and sodium starch glycolate are present, and compression of the granules to a tablet.

14. Method of preparing a pharmaceutical composition in the form of a tablet according to any one of the preceding claims, the method comprising:
a. blending edoxaban, or a salt thereof or a hydrate of said edoxaban or edoxaban salt, preferably edoxaban tosylate monohydrate, with lactose, preferably lactose monohydrate, and optionally a disintegrant selected from crospovidone and sodium starch glycolate to produce a blend;
b. wet granulating the blend of a. with a binder, preferably hydroxypropylcellulose, to produce granules;
c. blending the granules of b. with one or more of crospovidone and sodium starch glycolate, such that both crospovidone and sodium starch glycolate are present from steps a. and/or c., and optionally an additional filler, preferably pregelatinized starch, and optionally a lubricant, preferably magnesium stearate;
d. optionally lubricating the granules of c. by mixing with a lubricant, preferably magnesium stearate, such that at least one lubricant is present from steps c. and/or d.; and
e. compression of the granules to a tablet.

15. Method of preparing a pharmaceutical composition in the form of a tablet according to the preceding claim, wherein the method comprises additionally coating the tablet with a film coating, preferably comprising hydroxypropyl methyl cellulose (HPMC, hypromellose) and/or polyethylene glycol (PEG, Macrogol).
